# EUROPÄISCHE PATENTSCHRIFT

(11) **EP 0 856 294 B1**
(45) Veröffentlichungstag und Bekanntmachung des Hinweises auf die Patenterteilung: **08.08.2001**
(21) Anmeldenummer: 97116001.5
(22) Anmeldetag: 15.09.1997
(51) Int. Cl.: A61B 19/04

(54) **Gegenstand aus einem flexiblen Kunststoff**
Article fabricated from flexible synthetic material
Article en matière synthétique flexible

(30) Priorität: 12.09.1996 AT 162096
(43) Veröffentlichungstag der Anmeldung: 05.08.1998
(73) Patentinhaber: Semperit Aktiengesellschaft Holding, 1031 Wien (AT)
(72) Erfinder: Schaller, Raimund, Dipl.-Ing. Dr., 2632 Wimpassing (AT)
(74) Vertreter: Secklehner, Günter, Dr.

(56) Entgegenhaltungen:
- WO-A-92/13497
- WO-A-93/06996
- WO-A-95/17107

## Beschreibung

Die Erfindung beschreibt einen Gegenstand aus einem flexiblen Gummi und/oder Kunststoff sowie ein Verfahren zum Herstellen desselben, wie sie im Oberbegriff der Ansprüche 1 und 25 gekennzeichnet sind.

Viele an der Oberfläche unbehandelte, insbesondere flexible Gegenstände aus Kunststoff und Gummi, weisen einen relativ großen Reibungswiderstand gegenüber menschlicher Haut auf. So sind zum Beispiel unbehandelte Gummihandschuhe kaum anzuziehen. Um das Anziehen dieser Handschuhe zu erleichtern, wird konventioneller Weise Handschuhpuder (z.B. Stärkepuder, Talkum, usw.) als Gleitmittel auf der Innenschicht des Handschuhes aufgebracht. Die Verwendung von Puder ist jedoch insbesonders im medizinischen Bereich problematisch, da Puderreste, falls sie in offene Wunden gelangen, zur Granulombildung führen können. Ein weiterer wichtiger Anwendungsbereich für puderfreie Produkte liegt in der Elektronikindustrie.

Eine klassische Methode, Gegenstände aus Gummi und im speziellen Handschuhe gegenüber der menschlichen Haut ohne Verwendung von Puder schlüpfrig zu machen, besteht in der Oberflächenchlorierung. Hierbei wird der Gegenstand mit wäßrigen, chlorgashaltigen Medien behandelt und anschließend gewaschen. Dabei wird vor allem eine gute Gleitfähigkeit gegenüber der trockenen menschlichen Haut erreicht, jedoch wird durch diese Oberflächenchlorierung die Polymerstruktur künstlich gealtert und es kommt so zu einer deutlichen Verschlechterung der physikalischen und chemischen Eigenschaften des Gegenstandes (Festigkeit, Reißdehnung, Wasserquellung, Lagerfähigkeit, etc.). Derartig oberflächenbehandelte Handschuhe sind jedoch mit nassen Händen meist auch nur schwierig anziehbar.

Eine alternative Oberflächenbehandlung stellt die Beschichtung mit Hydrogelen dar. Einige solche Hydrogele sind schon seit langem bekannt. Dies sind z.B. Polyurethane, Polyvinylpyrrolidon, Polyhydroxyethylacrylat oder -metacrylat, Polyhydroxypropylacrylat oder -metacrylate und Copolymere miteinander oder mit Acryl- oder Metacrylsäure, Acryl- oder Metacrylester oder Vinylpyridin.

Eine derartige Beschichtung ist aus der US 3,813,695 A bekannt, die einen getauchten Gummihandschuh beschreibt, der an der Innenseite mit einem Hydrogelpolymer, wie z.B. Polyvinylpyrrolidon, Polyhydroxyethylacrylat oder -metacrylat, Polyhydroxypropylacrylat oder -metacrylat und Copolymere dieser untereinander oder mit Acryl- oder Metacrylsäure. Das bevorzugte Hydrogelpolymer ist ein Copolymer von 2-Hydroxyethylmetacrylat mit Metacrylsäure oder mit 2-Ethylhexylacrylat oder einem ternärem Copolymer von 2-Hydroxyethylmetacrylat, Metacrylsäure und 2-Ethylhexylacrylat.

Ein bekanntes Herstellungsverfahren ist aus der US 4,482,577 A bekannt. Bei diesem wird die Beschichtung eines flexiblen, vulkanisierten Operationshandschuhs mit einem hydrophilen Polymer geoffenbart, wodurch die Aufbringung von Puder an der Handschuhinnenseite unterlassen werden kann. Das hierbei verwendete Copolymer besteht aus einer Mischung von 2-Hydroxyethylmetacrylat und 2-Ethylhexylacrylat.

Ein anderes Verfahren gemäß US 4,100,309 A legt die Aufbringung einer gleitfähigen Schicht aus einem Polyurethan-Polyvinylpyrrolidon-Komplex offen. Üblicherweise wird bei diesen Produkten bzw. Verfahren das Hydropolymer gemeinsam mit einem Härtungsmittel in gelöster Form auf das Produkt aufgebracht, anschließend das Lösungsmittel durch Trocknung entfernt, wobei die Polymerschicht ausgehärtet und so eine Hydrogelschicht ausgebildet wird.

Weitere bekannte Lösungen offenbaren den Einsatz von Vinylidenhalogenid- bzw. Vinylhalogenidlatices zur Herstellung einer Gleitschicht auf Gummiartikel, wie z.B. US 5,069,965 A, wo die Gleitfähigkeit meist chlorhaltiger Polymere ausgenutzt wird.

Der Einsatz von diversen Latexgemischen für eine Gleitschichtherstellung ist aus der DE 26 28 059 C und der US 4,082,826 A bekannt. Hier kommen insbesondere Gemische aus mindestens zwei Latextypen zum Einsatz, wobei ein Latextyp mit einer hohen Dehnung als Haftvermittler und ein Latextyp mit hoher Härte bzw. geringer Dehnung zum Erhalt einer ausreichenden Gleitfähigkeit dient. Diese Rezepturen sind üblicherweise mehr oder minder gute Kompromisse zwischen Gleitfähigkeit, ausreichender Filmflexibilität und Haftung des Films am Gummiprodukt. Insbesondere beim Einsatz der Gleitschicht bei sehr flexiblen und hoch dehnbaren Gummiartikeln gibt es hier oft Probleme, daß die eingesetzten Gleitschichten entweder beim Dehnen sich vom Untergrund zu lösen beginnen bzw. die Gleitfähigkeit in der Praxis nicht befriedigend ist.

In dem Verfahren nach der EP 0 681 912 A2 wird die Gleitschicht aus einem Copolymerlatex gebildet, welcher mit Hilfe eines Koagulationsschrittes auf die Trägerschicht fixiert wird. Der Latex besteht aus einem Copolymeren, das einen wesentlichen Anteil an hydrophilen Monomerbausteinen enthält.

Einen anderen Weg zur Erzeugung von Hautschlüpfrigkeit bei puderfreien medizinischen Naturlatexhandschuhen wird in der US 4,143,109 A beschrieben. Dort werden in einer Trägerschicht gebundene Maisstärke- oder Polyethylenteilchen eingelagert, wobei die Trägerschicht dünner als der Teilchendurchmesser ist und die Teilchen aus der Oberfläche herausragen. Die so gebundenen Puderpartikel bewirken eine ausreichende Gleitfähigkeit der Handschuhinnenseite. Probleme ergeben sich bei dieser Lösung zur Erreichung von Hautschlüpfrigkeit bei größerer Dehnung der Produkte, was beim Anziehen von medizinischen Latexhandschuhen unvermeidlich ist: Die oberflächlich gebundenen Partikel lösen sich leicht ab und die Puderfreiheit ist nicht mehr gewährleistet.

Aus der WO 93/06996 A sind Gegenstände bekannt, die zur Anlage an der menschlichen Haut ausgebildet sind, beispielsweise Handschuhe, welche aus einer Elastomerschicht und einer darauf angeordneten Polymerschicht bestehen. Die Polymerschicht kann z.B. durch Copolymere von Vinylmethylether mit Maleinsäure oder durch Alkylmonoester von Poly(Methylvinylether - maleinsäure) gebildet werden. Diese Polymerschicht weist eine Rauheit auf, wobei die gemittelte Rauhtiefe im Bereich zwischen 0,55 µm bis 2,92 µm beträgt.

Alle oben beschriebenen puderfreien Artikel sind nur unter genauestem Einhalten der zugehörigen Prozeßbedingungen und Materialrezepturen mit befriedigenden Produkteigenschaften herstellbar. Während eine gute Gleitfähigkeit der Oberflächen gegenüber trockener Haut meist leicht erreicht wird, gibt es sehr oft Schwierigkeiten in der Reproduzierbarkeit hinsichtlich Naßgleitfähigkeit. Insbesondere bei dünnwandigen flexiblen Artikeln, welche auch unter hohen Dehnungen gute Gleitfähigkeiten aufweisen müssen, bedarf es oft jahrelanger Feinoptimierung seitens der Hersteller, welche meist Kompromisse seitens diverser Produkteigenschaften eingehen müssen. So wird vielfach trotz Einsatz von polymeren Gleitschichthydrogelen zusätzlich die Oberfläche leicht chloriert. Andere Hersteller verwenden trotz Polymergleitschichten zusätzlich geringe Mengen von Handschuhpuder. Wieder andere Produzenten sehen sich mit laufenden Schwankungen hinsichtlich der Naßgleitfähigkeit ihrer Produkte konfrontiert.

Der vorliegenden Erfindung liegt die Aufgabe zugrunde, Gegenstände aus Kunststoff und/oder Gummi herzustellen, die eine gute Schlüpfrigkeit gegenüber der menschlichen, insbesondere nassen Haut aufweisen und ein Verfahren zu schaffen, welches eine ungestörte Reproduzierbarkeit aufweist.

Diese Aufgabe der Erfindung wird durch den Gegenstand nach Anspruch 1 gelöst. Von Vorteil ist hierbei, daß die flexiblen Produkte mit der erhabenen Netzstruktur und den vertieften Flächen und die dadurch gebildete, leicht gerauhte Oberfläche eine wesentlich bessere Schlüpfrigkeit vor allem gegenüber der nassen menschlichen Haut aufweisen als glatte Gleitschichten. Dies gilt insbesondere für die oft schwer erreichbare Naßgleitfähigkeit. Ein weiterer Vorteil der vorliegenden Erfindung ist, daß durch die Vermeidung von okklusivem Hautkontakt der Tragekomfort, der Abtransport und gegebenenfalls die Sorption von Schweiß wesentlich verbessert wird. Wesentlich ist auch, daß durch die rigorose Verringerung der Kontaktfläche zwischen Haut und Polymer in vielen Fällen eine wesentlich verbesserte Hautverträglichkeit zu erwarten ist. Zusätzlich werden allergische Reaktionen verringert und es entsteht kein Engegefühl nach dem Überziehen der Produkte. Vorteilhaft ist, daß die erfindungsgemäß gerauhte Oberflächenmorphologie ohne zusätzliche mechanische oder chemische Behandlung, wie z.B. Präge- oder Chlorierverfahren, und z.B. auch ohne Zusatz eines Gleitmittels, z.B. Puders oder Silikon, hergestellt werden kann. Es kann dadurch eine gute Gleitfähigkeit, auch mit weniger optimalen Gleitschichtpolymeren, erreicht werden. Bei Einhaltung einer gerauhten Oberflächenstruktur kann eine gute Schlüpfrigkeit sowohl im nassen als auch im trockenen Zustand erreicht werden.

Vorteilhaft ist auch eine weitere Ausgestaltung nach Anspruch 2, da durch die Fülle der dadurch gebildeten Mikrohohlräume das Reibungspotential bzw. die Berührungs-fläche zwischen der menschlichen Haut und dem Gegenstand erheblich reduziert werden kann.

Durch die vorteilhafte Ausführungsvariante nach Anspruch 3 wird sichergestellt, daß ein Großteil der Gleitschicht durch die netzartige Struktur auch im angezogenen Zustand, in dem der Gegenstand leicht gedehnt oder gespannt ist, von der Haut distanziert gehalten ist, sodaß trotz der Aufrauhung einerseits für den Benutzer nicht das Gefühl entsteht, daß der Gegenstand auf der Haut reibt und andererseits die Beweglichkeit der Hand, beispielsweise der Finger, nicht behindert wird, da die Gleitschicht bzw. die Teilfläche der Gleitschicht nicht an der nassen Haut anhaftet.

Ein angenehmes Gefühl auf der Haut hinterläßt ein Gegenstand, wenn die Gleitschicht gemäß Anspruch 4 ausgebildet ist.

Eine gute Hautverträglichkeit für Gegenstände wird bei einer Ausführungsform nach Anspruch 5 erreicht.

Durch die Verwendung der im Anspruch 6 und 7 angegebenen Materialien, kann eine bei den durch entsprechende Nachbehandlung durch die chemischen und/oder physikalischen Reaktionen hervorgerufene Oberflächenrauheit einfach erzeugt werden.

Durch die weitere Ausgestaltung nach Anspruch 8 kann der Schweißhaushalt an der Hautoberfläche im Bereich der gerauhten Oberflächen in den Teilbereichen besser gesteuert werden, da der Handschuh eine höhere Menge an Feuchtigkeit aufnehmen kann.

Eine gute Verbindung mit der Gleitschicht in den Teilbereichen wird durch die Weiterbildung nach Anspruch 9 erzielt.

Es ist auch eine Ausbildung nach Anspruch 10 möglich, wodurch die Naßschlüpfrigkeit der Gegenstände zusätzlich verbessert werden kann.

Bevorzugt ist eine Ausgestaltung nach Anspruch 11, da dadurch eine so dünne, die Gleitfähigkeit zusätzlich erhöhende Schicht geschaffen wird, bei der gleichzeitig ein Verkleben der Vertiefungen verhindert ist.

Von Vorteil ist bei der Ausgestaltung nach Anspruch 12, 13 und/oder 14, daß damit die Schlüpfrigkeit und die Gleiteigenschaften sowie eine entsprechende Rauheit, beispielsweise das Erfassen von Arbeitswerkzeugen bzw. Gegenständen bei der Montage erzielt werden können.

Vorteilhaft ist eine Weiterbildung nach Anspruch 15, da dadurch extrem dünne und extrem dehnbare sowie stark belastbare Gegenstände herstellbar sind.

Die Qualität der Gegenstände kann durch die Anwendung der Merkmale nach den Ansprüchen 16 oder 17 erheblich verbessert werden.

Eine hohe Elastizität und Anschmiegsamkeit des Gegenstandes ermöglicht eine Ausführungsvariante nach Anspruch 18.

Vorteilhaft ist es für die Widerstandsfähigkeit der Trägerschicht, diese nach Anspruch 19 auszubilden.

Vorteilhaft ist eine Ausführungsvariante nach Anspruch 20 oder 21, da dadurch die Wirkung der Rauheit im Bereich der Trägerschicht noch verstärkt bzw. in jenen Bereichen, in denen keine Aufrauhung erfolgt, zumindest eine bedingte Verbesserung der Gleiteigenschaften erzielt wird.

Bevorzugte Ausgestaltungen des Gegenstandes sind in den Ansprüchen 22 und 23 gekennzeichnet.

Eine weitere Ausführungsform des Gegenstandes ist in Anspruch 24 angegeben.

Die Erfindung umfaßt auch ein Verfahren zur Herstellung eines flexiblen Gegenstandes, insbesondere eines Handschuhes, wie es im Oberbegriff des Anspruches 25 beschrieben ist.

Durch die Maßnahmen im Kennzeichenteil des Anspruches 25 wird die erfindungsgemäße Aufgabe gelöst und wird durch die einzelnen aufeinanderfolgenden Maßnahmen eine Verwerfung in der Oberfläche der Gleitschicht und damit das Einsinken der die Gestaltsabweichungen darstellenden Vertiefungen gegenüber den in der ursprünglichen Höhe verbleibenden Stegen der netzartigen Struktur gebildet, wodurch ein höherer Tragekomfort durch die höhere Elastizität, vor allem bei Fingerbewegungen erzielt und die Gleitfähigkeit des Gegenstandes auf der menschlichen Haut, vor allem dann, wenn diese naß ist, zusätzlich verbessert wird. Darüber hinaus bedürfen die während des Herstellungsprozesses hergestellten Aufrauhungen keiner zwingenden zusätzlichen Behandlung, wie einer nachfolgenden Chlorierung oder einem Gleitmittelauftrag oder dgl..

Vorteilhaft in bezug auf die Wirtschaftlichkeit des Verfahrens und die Qualität der danach hergestellten Produkte ist es auch, das Verfahren nach den Ansprüchen 26, 27 oder 28 auszubilden.

Durch das Vorgehen nach Anspruch 29 kann durch die sich im Zuge des Trockenvorganges einstellende aufgerauhte Struktur an der Oberseite der Gleitfläche bzw. die netzartige Struktur von Stegen, die um das Ausmaß zwischen 0,5 µm und 100 µm über die beim Entwässern eingesunkenen Gestaltabweichungen vorragen, ein Stützgitter erzeugt werden, welches sich auf der Oberfläche der Haut abstützt und die Berührungs-fläche zwischen Haut und Gegenstand verringert, ohne daß ein unangenehmer Kratzeffekt, wie er bei zu hoher Rauhigkeit der Oberfläche feststellbar ist, auftritt.

Durch die hohe Temperatur der die Infrarotstrahlung abgebenden Strahlungskörper gemäß Anspruch 30 wird eine rasche Fixierung dieser Oberflächenverwerfungen ermöglicht, ohne daß es zu einer Rückbildung der netzartigen Struktur kommt.

Durch die weitere Vorgangsweise nach Anspruch 31 wird erreicht, daß die chemische Reaktion im gesamten Gegenstand, sowohl in der Tragschicht als auch in der Gleitschicht abgeschlossen und eine sichere Verbindung zwischen diesen Schichten erzielt wird.

Durch die kurze Zeitspanne gemäß Anspruch 32 und gegebenenfalls die Maßnahme nach Anspruch 33 wird verhindert, daß die durch das Entwässern entstehenden eingesunkenen Gestaltsabweichungen sich durch eine gleichmäßige Verteilung der z.B. gelartigen Flüssigkeit über die Schicht wieder ausnivellieren können, bevor sie endgültig fixiert sind.

Eine gute Haftung zwischen der Trägerschicht und der Gleitschicht wird durch ein Vorgehen nach den Ansprüchen 34 bis 36 erreicht.

Eine rasche Entwässerung der Gleitschicht und ein damit einhergehendes stärkeres Einsinken der Gestaltsaufweichung gegenüber der netzartigen Struktur wird durch die Maßnahme nach Anspruch 37 erzielt.

Die Verwendung von Polymeren für die Gleitschicht gemäß Anspruch 38 erhöht die Hautverträglichkeit und vereinfacht die Herstellung der gegenüber der netzartigen Struktur abgesenkten Gestaltsabweichungen.

Von Vorteil ist eine Zusammensetzung der Dispersion nach einem der Ansprüche 39 bis 41, da dadurch die Bildung der netzartigen Struktur und der die Gestaltsabweichungen bildenden Vertiefungen begünstigt wird.

Die Herstellung der Rauheit in der Trägerschicht und/oder der Gleitschicht bzw. deren Teilflächen kann durch das Vorgehen nach den Ansprüchen 42 und 43 verbessert werden.

Eine weitere Verbesserung der Gegenstände wird durch die Maßnahmen nach Anspruch 44 erreicht.

Schließlich ist in Anspruch 45 noch eine weitere Ausführungsvariante des Verfahrens angegeben.

Die Erfindung wird im nachfolgenden anhand der in den Zeichnungen dargestellten Ausführungsbeispiele näher erläutert.

Es zeigen:
- Fig. 1: einen Teilbereich eines erfindungsgemäßen Gegenstandes mit einer Gleitfläche und einer entsprechenden Rauheit, geschnitten und in vereinfachter, schaubildlicher, schematischen Darstellung;
- Fig. 2: einen erfindungsgemäßen Gegenstand in der Art eines Handschuhes, in Draufsicht, teilweise geschnitten;
- Fig. 3: den Gegenstand nach Fig. 2 in Stirnansicht, geschnitten, gemäß den Linien III - III in Fig. 2 und vereinfachter, schematischer Darstellung;
- Fig. 4: einen Teil des erfindungsgemäßen Verfahrensablaufes zur Herstellung eines Gegenstandes in vereinfachter, schematischer Darstellung;
- Fig. 5: einen weiteren Teil des erfindungsgemäßen Verfahrensablaufes zur Herstellung eines Gegenstandes in vereinfachter, schematischer Darstellung;
- Fig. 6: eine Stirnansicht einer Porzellanform, geschnitten mit den darauf aufgebrachten Lagen zur Herstellung eines durch einen Handschuh gebildeten, erfindungsgemäßen Gegenstandes, in vereinfachter, schematischer Darstellung;
- Fig. 7: eine Draufsicht auf die Oberfläche der Gleitschicht eines erfindungsgemäßen Gegenstandes in mit einem Elektronenmikroskop stark vergrößerter Darstellung.

Einführend sei festgehalten, daß in den unterschiedlich beschriebenen Ausführungsbeispielen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Weiters können auch Einzelmerkmale aus den gezeigten unterschiedlichen Ausführungsbeispielen für sich eigenständige, erfindungsgemäße Lösungen darstellen.

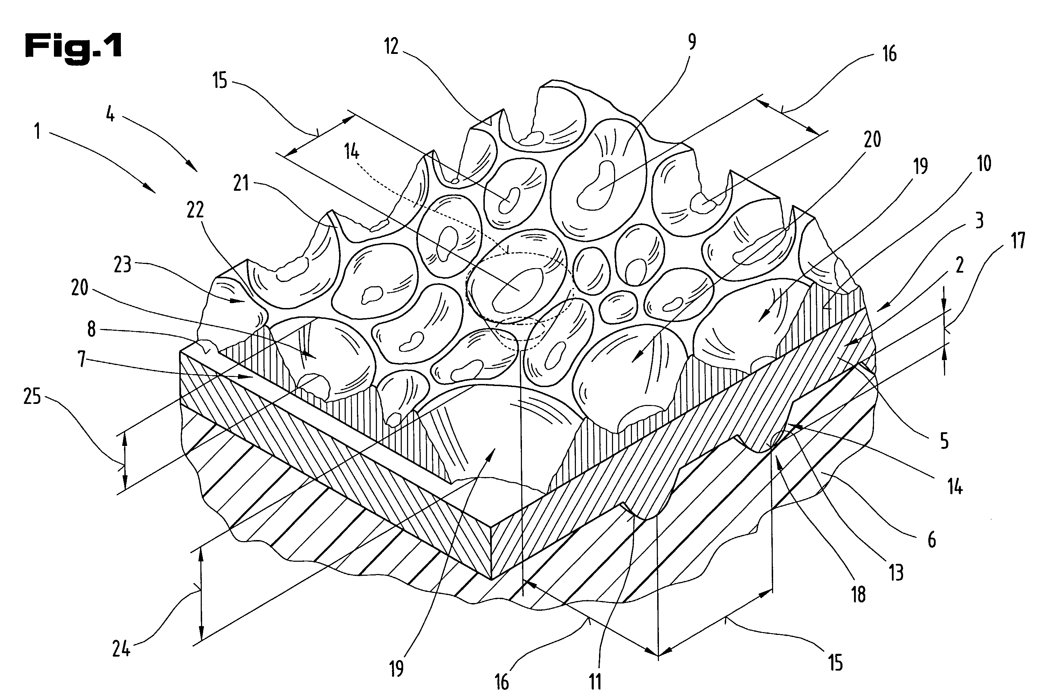

In der Fig. 1 ist ein erfindungsgemäßer, flexibler Gegenstand 1, der bevorzugt aus einem Latex-Produkt 2 gebildet ist, gezeigt. Dieser besteht aus einer Trägerschicht 3, die, wie dies bei der Herstellung von dünnwandigen Gummi- und Kunststoffartikeln üblicherweise der Fall ist, bevorzugt durch ein mehrmaliges Tauchverfahren gebildet wird. Bei diesen Gegenständen 1 kann es sich um Handschuhe 4, beispielsweise medizinische Operations- und Untersuchungshandschuhe, Katheder, Kondome, Fingerlinge, Badehauben, Schwimmflossen oder Schutzhandschuhe für die Arbeit in Reinraumbereichen handeln. Dünnwandige, insbesondere elastische Gummi- und/oder Kunststoffartikeln, wie z.B. der Handschuh 4 werden vorzugsweise durch Tauchen einer Form in ein Latex-Produkt 2 erhalten, wobei dieses Latex-Produkt 2 sowohl auf Naturlatex als auch auf Syntheselatex basieren kann. Die verwendeten Latex-Produkte 2 können sowohl unvernetzt als auch im vorvernetzten Zustand vorliegen. Im Falle eines unvernetzten oder nur partiell vorvernetzten Latex-Produktes 2 muß die erhaltene Gummischicht noch an der Tauchform vulkanisiert werden, wodurch eine Trägerschicht 3 gebildet wird.

Vorzugsweise wird ein Latex-Produkt 2 verwendet, welches zumindest ein vernetztes oder größtenteils vorvernetztes Polymer 5 enthält. Dieses Latex-Produkt 2 wird auf eine Tauchform 6, welche die Gestalt des Endproduktes vorgibt, aufgebracht und koaguliert und erzeugt so eine dünne Schicht, nämlich die Trägerschicht 3, aus elastischem, relativ resistentem Gummimaterial. Das Latex-Produkt 2 enthält die üblichen Compoundierungszutaten, wie beispielsweise Schwefel, Zinkoxid, organische Beschleuniger (u.a. Zinksalze von Dithiocarbamaten, Thiurame, Thioharnstoff, etc.), Stabilisatoren, Wachse, Alterungsschutzmittel, Viskositätsregler, Füllstoffe, Farben, usw.. Als Latex-Produkt 2 für die Trägerschicht 3 kann sowohl Naturkautschuk, als auch Synthesekautschuk, der für die Anwendung in Tauchverfahren geeignet ist, verwendet werden. Von den natürlichen und synthetischen Latices werden vorzugsweise Naturkautschuk, Polychloropren, synthetisches Polyisopren, Nitrilbutadien- und Styrolbutadienkautschuk bzw. eine Mischung dieser Polymere verwendet.

Selbstverständlich können alle anderen dem Fachmann aus dem Stand der Technik bekannten Herstellungsverfahren für die Trägerschicht 3 verwendet werden.

Es ist seit langem in der Fachwelt bekannt, daß, um reproduzierbare Schichten aus Latex-Produkten auf einer Form herstellen zu können, auf die Form, beispielsweise eine mit entsprechenden Aufrauhungen versehene Keramikform, ein Koagulant aufgebracht wird. Dazu wird üblicherweise die Form in ein Becken bzw. einen Behälter, in welchen der Koagulant in flüssiger Form vorhanden ist, eingetaucht. Dieser Koagulant kann jede aus dem Stand der Technik bekannte Zusammensetzung aufweisen, wie beispielsweise Alkohollösungen von Kalziumsalzen oder dgl. Das Koagulant enthält auch ein Trennmittel, wie beispielsweise Talkum oder Kalziumcarbonat, das, wenn es säurelöslich ist, bei nachfolgenden Säurebehandlungen aus den Oberflächenschichten herausgelöst werden kann, sodaß ein puderfreier Handschuh entsteht. Anschließend wird der Koagulant getrocknet.

Daraufhin wird die Form mit dem bevorzugt getrockneten Koagulant in einen Behälter, in dem das Latex-Produkt 2 als Dispersion bzw. Flüssigkeit vorrätig gehalten wird, eingetaucht, sodaß eine durchschnittliche Schichtdicke von 100 µm bis 300 µm erreicht wird. Bevorzugt ist es auch möglich, die Form mehrmals in das Latex-Produkt 2 einzutauchen, um die Schichtdicke zu erhöhen, wobei zwischendurch ein kurzes Antrocknen der Latex-Schicht erfolgen kann.

Durch die chemische Reaktion des Latex-Produktes 2 mit dem Koagulant härtet das flüssig aufgetragene Latex-Produkt 2 aus. Bevorzugt wird unmittelbar nach dem Aufbringen des Latex-Produktes 2 auf die Form diese mit Heißluft kurz angetrocknet, so- daß die Oberfläche der Trägerschicht 3 fest wird bzw. geliert, wobei diese beispielsweise in einem Wärmeofen oder einem Behälter, in dem Warmluft mit einer Temperatur zwischen 70°C und 140°C hindurchgeführt wird, 15 sec. bis 60 sec. getrocknet wird.

Nach dieser Zwischentrocknung wird dann auf diese Trägerschicht 3 zumindest in einem Teilbereich 7 einer Oberseite 8 der Trägerschicht 3 eine Gleitschicht 9 aus polymeren Material 10 im Zuge des vorhin umrissenen Tauchprozesses durch Tauchen oder Sprühen in einem oder mehreren Schritten auf die angetrocknete Trägerschicht 3 aufgetragen. Die Schichtdicke dieser Gleitschicht 9 kann entsprechend den unterschiedlichen Erfordernissen, insbesondere der danach gewünschten Rauhtiefe festgelegt werden und beträgt zwischen 2 µm und 80 µm, bevorzugt 2 µm bis 30 µm.

Diese Gleitschicht 9 kann durch ein Gemisch aus mehreren polymeren Materialien 10, wie beispielsweise wässerige Polyurethandisperison gebildet sein. Dabei kann je nach Einsatzzweck ein Polyacrylat, ein Polysiloxan, ein Polymethacrylat, ein carboxyliertes Styrol-Butadien Copolymer, ein Polyvinylpyrollidon, ein kationisches Polyurethan, z.B. mit einem Molekulargewicht von mindestens 100 000, ebenso verwendet werden, wie die nichtionischen oder anionischen Ausführungen dieser vorgenannten Materialien. Diese wässerigen Disperisonen und beliebige Mischungen davon bilden Schichten bzw. Filme mit guten mechanischen Grundeigenschaften und weisen ähnliche Dehnungseigenschaften auf wie die Trägerschicht 3. Selbstverständlich kann diese Gleitschicht 9 auch aus einem Gemisch von mehreren unterschiedlichen polymeren Materialien 10 in einer wässerigen Dispersion gebildet sein. So wird bevorzugt die Dispersion durch ein Gemisch aus 0 Gew% bis 30 Gew% Polyurethan-, 1 Gew% bis 40 Gew% Polyacrylat-, 1 Gew% bis 20 Gew% Polysiloxandispersion und 0 Gew% bis 10 Gew% Füllstoffe und der restliche Anteil aus Wasser gebildet. Als Füllstoffe können pulver- oder puderförmige Materialien, wie z.B. Kreide, Kalk, Gips, Siliciumdioxid und/oder Maisstärke, zur Anwendung kommen. Für die Herstellung der Gleitschicht 9 beim erfindungsgemäßen Gegenstand 1 bzw. beim Handschuh 4 können aber auch andere Mischungen, wie beispielsweise 5 Gew% bis 15 Gew% Polyurethan-, 1 Gew% bis 8 Gew% Polyacrylat-, 2 Gew% bis 6 Gew% Polysiloxandispersion und 4 Gew% bis 6 Gew% Füllstoffe und der Rest Wasser ebenso wie die nachfolgend in den einzelnen Klammern angegebenen Mischungsverhältnisse, bei welchen die einzelnen Anteile an Gew% der Dispersionen aus Polyurethan, Polyacrylat, Polysiloxan und Füllstoffe durch Schrägstriche getrennt sind, wobei der restliche Anteil auf 100 Gew% durch Hinzumischung von Wasser gebildet ist, verwendet werden. Der Trockengehalt der Dispersionen ist vom Fachmann festlegbar, wobei der Feststoffgehalt bei Polyurethan zwischen 30 % und 50 %, bei Polyacrylat 30 % bis 50 % und bei Polysiloxan 20 % bis 40 % betragen kann. Bei diesen Gemischen handelt es sich um folgende (2-7/4-10/3-12/0-5), (0-10/2-6/3-10/3-7), (8-18/5-15/4-7/5-10), (12-22/12-26/16-20/0-4), (17-26/18-32/10-14/2-6), (24-30/28-40/15-20/6-9), (24-30/25-35/5-10/3-7), (21-27/4-9/1-4/6-8), (21-27/12-22/3-9/4-7), (21-27/28-36/12-20/2-7), (9-12/1-3/2-6/5-9), (12-22/4-9/1-4/0-3), (17-26/5-11/7-10/0-4), (2-7/12-22/14-20/3-8), (5-15/28-36/14-20/5-10), (0-10/24-29/9-16/5-8).

Im speziellen Fall der Herstellung von Tauchartikel, wie beim Gegenstand 1, wird deren Oberfläche üblicherweise in die Tauchformseite und die Tauchbadseite unterteilt. Die Tauchformseite ist im vorliegenden Ausführungsbeispiel als Unterseite 11 bezeichnet und liegt an der Tauchform 6 an, die beispielsweise aus Porzellan, Kunststoff oder ähnlichem bestehen kann.

Die Tauchbadseite wird entweder durch die Oberseite 8 der Trägerschicht 3 oder die Oberfläche 12 der Gleitschicht 9 gebildet. Im vorliegenden Fall ist eine Oberfläche 13 der Tauchform 6 mit bevorzugt regelmäßig wiederkehrenden Gestaltsabweichungen 14 versehen. Diese werden durch Gestaltsabweichungen 14, beispielsweise Vertiefungen in der Oberfläche 13 oder durch über die Oberfläche 13 vorragende Erhebungen, gebildet. Ein Verhältnis von Abständen 15, 16 zu einer Tiefe 17 derselben liegt zwischen 100 : 1 und 5 : 1. Damit entspricht die Rauheit der Unterseite 11 des Gegenstandes 1 der Morphologie der Tauchform 6. Die Tiefe beträgt dabei zwischen 0,5 µm und 100 µm, bevorzugt zwischen 1 µm und 40 µm.

Aufgrund der geringen Tiefe 17 der Gestaltsabweichungen 14 sei bereits an dieser Stelle ausdrücklich festgehalten, daß in allen Darstellungen zur verbesserten, schematischen Darstellung der Rauhtiefe diese Gestaltsabweichungen völlig unproportional, äußerst stark vergrößert dargestellt sind, um die Wirkungen und auch die Anordnung dieser Gestaltsabweichungen 14 und auch der anderen Gestaltsabweichungen überhaupt darstellen und erläutern zu können.

Wird, wie nachfolgend noch im Detail erläutert wird, sichergestellt, daß eine Schichtdicke der Trägerschicht 3 sowie der auf dieser aufgebrachten Gleitschicht 9 in den Teilbereichen 7 über einen Großteil des Gegenstandes 1 gleichgehalten wird, so bildet sich auch eine entsprechende Rauheit auf der der Tauchbadseite zugewandten Oberfläche 12 der Gleitschicht 9 aus. Diese Tauchbadseite bildet üblicherweise die Innenseite des Produkts, wenn es sich beispielsweise um hüllenförmige Gegenstände 1 bzw. dünnwandige Gummiprodukte handelt, da beim Abziehen der Gegenstände 1 von der Tauchform 6 die Produkte gewendet werden. Üblicherweise ist die Oberfläche 12 jene Seite des Gegenstandes 1, die mit der Haut des Benutzers in Berührung kommt und dem entsprechend ausgebildet sein soll, um Irritationen der Haut bzw. die Schweißabsonderung ohne Störung der Haut zu ermöglichen. Beim vorliegenden Gegenstand 1 ist vorgesehen, daß entweder die Tauchformseite oder Tauchbadseite oder beide mit einer entsprechenden Rauheit ausgestattet werden können. Dementsprechend ist es auch möglich, daß auf einer oder beiden Seiten in Teilbereichen 7, 18 aber selbstverständlich auch über die gesamte Oberseite 8 bzw. Unterseite 11, eine Gleitschicht 9 angeordnet sein kann.

Im Rahmen der vorliegenden Erfindung ist es aber auch möglich, daß sowohl auf der Oberseite 8 als auch auf der Unterseite 11 eine Gleitschicht 9 angeordnet ist, wobei dies dadurch erfolgen kann, daß nach Herstellen der Trägerschicht 3 der Gegenstand 1 von der Tauchform 6 abgezogen und in gewendeter Lage, also mit der Oberseite 8 auf der Tauchform 6 anliegend, auf die gleiche oder eine andere Tauchform aufgezogen wird, um danach auch auf die Unterseite 11, die nun auf der Tauchbadseite angeordnet ist, ebenfalls eine Gleitschicht 9 aufzubringen.

Durch zumindest in Teilbereichen 7 auf der Oberseite 8 der Trägerschicht 3 angeordnete Gestaltsabweichungen 19, 20 in der Gleitschicht 9 kann der Schweißhaushalt geregelt und der Tragekomfort erhöht werden.

Die Kontaktfläche zwischen Haut und Gleitschicht 9 bzw. dem polymeren Material 10 soll möglichst gering gehalten werden, um ein Festkleben an der z.B. nassen menschlichen Haut zu verhindern, wodurch unter anderem die Gefahr von Hautirritationen und unter Umständen allergischen Reaktionen minimiert werden. Des weiteren wird durch die erfindungsgemäß ausgebildete Oberfläche 12 eine gute Gleitfähigkeit der flexiblen Gegenstände 1 gegenüber der menschlichen Haut und daraus resultierend eine gute Anziehbarkeit im nassen und trockenen Zustand erreicht.

Aus der Darstellung in Fig. 1 ist weiters deutlich zu entnehmen, daß gegenüber den Gestaltsabweichungen 14 im Bereich der Unterseite 11 der Trägerschicht 3 in der Oberfläche 12 der Gleitschicht 9 dazu unterschiedliche Gestaltsabweichungen 19, 20 angeordnet sind. Diese Gestaltsabweichungen 19, 20 sind zwischen Stegen 21, 22 einer netzartigen Struktur 23 angeordnet bzw. zum größten Teil von diesen umgeben. Die Gestaltsabweichungen 19, 20 weisen gegenüber der Oberfläche 12 der Stege 21, 22 unterschiedliche Tiefen 24, 25 auf, wobei sich diese Tiefe 24, 25 über die Fläche der Gestaltsabweichungen 19, 20 ebenfalls verändern und gegebenenfalls bis zur Trägerschicht 3 reichen kann, wie dies beispielsweise bei der Gestaltsabweichung 19 gezeigt ist. Die Tiefe 24 dieser Gestaltsabweichung 19 entspricht damit der Gesamtschichtdicke der Gleitschicht 9, die zwischen 2 µm und 80 µm, bevorzugt 2 µm bis 30 µm, betragen kann.

Auch sind Abstände 15, 16 zwischen den einzelnen Gestaltsabweichungen 19, 20 stark unterschiedlich. Somit kann das Verhältnis der mittleren Abstände 15, 16 zwischen den einzelnen Gestaltsabweichungen 19, 20 zu ihrer Tiefe 24, 25 gegenüber der erhabenen, netzartigen Struktur 23 bzw. den Stegen 21, 22 zwischen 500 : 1 und 5 : 1 liegen. Die gemittelte Rauhtiefe beträgt beispielsweise zwischen 0,5 µm und 100 µm, bevorzugt zwischen 1 µm und 40 µm.

Wie weiters aus der Darstellung in Fig. 1 schematisch zu ersehen ist, sind zwischen 50 % und 90 %, bevorzugt 60 % bis 80 % der Teilfläche bzw. des Teilbereiches 7 durch Gestaltsabweichungen 19, 20 vertieft angeordnet. Bevorzugt sind zumindest 50 % der Gestaltsabweichungen 19, 20 in der Draufsicht auf die Gleitschicht 9 allseitig von der netzartigen Struktur 23, insbesondere den Stegen 21, 22 umgrenzt.

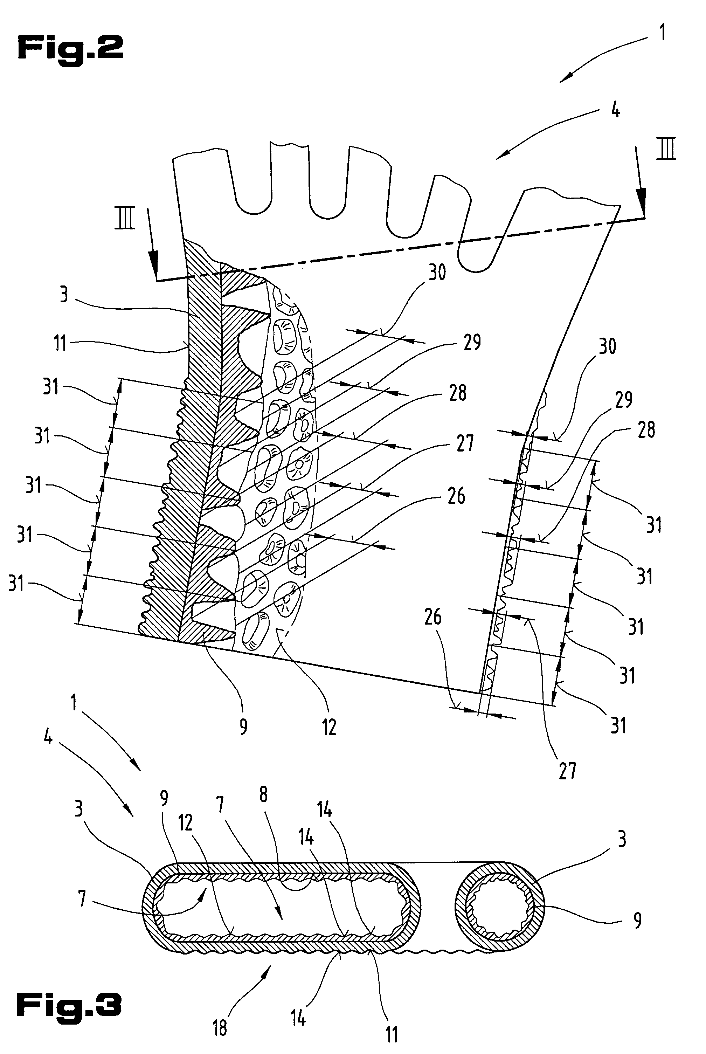

Weiters beträgt in bevorzugter Weise eine gemittelte Rauhtiefe der Gleitschicht 9 bzw. der Teilfläche des Teilbereiches 7 zwischen 0,5 µm und 100 µm, bevorzugt zwischen 1 µm und 40 µm. Diese gemittelte Rauhtiefe stellt das arithmetische Mittel aus Einzelrauhtiefen dar, die in fünf aneinandergrenzenden Einzelmeßstrecken gleicher Länge ermittelt werden, wie dies nachfolgend beim Ausführungsbeispiel in Fig. 2 noch näher erläutert werden wird. Dadurch wird erreicht, daß ein ziemlich einheitliches Haftungsvermögen bzw. Gleitvermögen zwischen den einzelnen Bereichen der Oberfläche 12 bzw. falls auch die Unterseite 11 mit einer Gleitschicht 9 beschichtet ist, auf der dieser vorgeschalteten Oberfläche erreicht wird, wodurch die Schlüpfrigkeit des Gegenstandes 1 und vor allem auch die Naßschlüpfrigkeit erheblich verbessert werden kann.

Unterschiedlich ist hierbei zu den bisher bekannten Verfahren, daß die erfindungsgemäßen Produkteigenschaften vorwiegend über die Rauhheit der Oberfläche 12 bzw. Unterseite 11 erreicht werden, wobei mit steigender gemittelter Rauhtiefe bis etwa 40 µ Rauhtiefe die Schlüpfrigkeit gegenüber der menschlichen Haut steigt und die Klebrigkeit der Polymeroberflächen abnimmt.

Wird die Oberfläche 12 bzw. Unterseite 11, welche die äußere Oberfläche des Handschuhs 4 bildet, weiter aufgerauht, tritt bei den meisten Produkten abhängig von Härte und Dehnungsmodul schrittweise ein subjektiv unangenehm reibendes Gefühl auf. Bei Rauhtiefen über 100 µm kommt es meist zu einer wesentlichen Erhöhung der Polymer-Polymerreibung und eventuell bei reibendem Kontakt mit menschlicher Haut zu mechanisch bedingten Irritationen. Die hier in ihrer Abhängigkeit von der gemittelten Rauhtiefe beschriebenen Oberflächeneigenschaften sind natürlich auch von Art und Herstellungstyp der Rauheit sowie von Polymer- und Produkttyp abhängig und müssen für jeden Polymer- und Produkttyp einzeln optimiert werden. Vorteilhaft ist dabei, daß durch die gemittelte Rauhtiefe, die über die Teilbereiche 7 bzw. 18 gleich ist, einzelne Irritationszonen in den Teilbereichen 7, 18, die den positiven Gesamteffekt der erfindungsgemäßen Lösung nachteilig beeinflussen können, grundsätzlich vermieden werden.

Es können in Gegenwart erfindungsgemäßer Oberflächenrauheiten weiters auch mit relativ weichen Polymeren (z.B. Polyacrylaten) gute Gleitfähigkeiten gegenüber der menschlichen Haut erreicht werden, wobei bei konventionell glatten Gleitschichten mit denselben Polymeren keine Hautschlüpfrigkeit vorhanden ist. Durch den Umstand, daß bei erfindungsgemäßen Oberflächenschichten weichere Polymere mit höheren Zugdehnungen eingesetzt werden können, verringern sich bei dünnwandigen Gegenständen 1 mit hoher Dehnfähigkeit die Haftungsprobleme der Oberflächenschicht beim Dehnen derselben. Des weiteren wird die Auswahl des Gleitschichtpolymers wesentlich vereinfacht, da der Haupteinflußfaktor für Tragekomfort und Schlüpfrigkeit erfindungsgemäß durch die Oberflächenmorphologie bestimmt wird.

Bei der Ausführungsform in den Fig. 2 und 3 ist die Ausgestaltung eines Gegenstandes 1 in Form eines puderfreien, z.B. medizinischen, Handschuhes 4 dargestellt.

Der in den Fig. 2 und 3 gezeigte Handschuh 4 ist in seiner von der Tauchform 6 abgezogenen Stellung gezeigt, wobei die bei der Produktion der Tauchbadseite zugewandte Oberfläche 12, die die Gleitschicht 9 aufnimmt, eine Innenseite des Handschuhs 4 bildet, während die bei der Herstellung des Handschuhs 4 der Tauchform 6 zugewandte Unterseite 11 die Außenseite des Handschuhs 4 bildet. Somit ist bei diesem Handschuh 4 auf der äußeren Oberfläche, mit der also durch den Benutzer des Handschuhs 4 Werkzeuge bzw. Bauteile erfaßt werden, eine entsprechende gemittelte Rauhtiefe vorhanden, die zwischen 0,5 µm und 100 µm, bevorzugt zwischen 1 µm und 40 µm beträgt und ein Abbild der gemittelten Rauhtiefe der Oberfläche der Tauchform 6 darstellt.

Um die gleiche Morphologie zu erzielen, wird wie bereits auch einleitend kurz aufgezeigt, die üblicherweise leicht rauhe Tauchform 6 aus Porzellan mit einer gemittelte Rauhtiefe zwischen 0,5 µm und 100 µm, bevorzugt 1µm bis 40 µm, verwendet, die in eine wäßrige Koagulationslösung, welche z.B. Kalziumnitrat, Polyethylenglykol und einen wasserlöslichen Silikontyp oder Kreide enthält, getaucht und dann kurz mit Heißluft getrocknet. Anschließend wird zur Aufbringung des Handschuhes 4 die Tauchform 6 in eine, z.B. vorvernetzte, Naturlatexmischung getaucht, aus der die Trägerschicht 3 gebildet wird.

Die gemittelte Rauhtiefe stellt das arithmetische Mittel aus Einzelrauhtiefen 26 bis 30 dar, die in fünf aneinandergrenzenden Einzelmeßstrecken gleicher Länge 31 ermittelt werden. Die gemittelte Rauhtiefe im Bereich der Oberfläche 13 stellt daher ein spiegelbildliches Abbild zur gemittelten Rauhtiefe der Tauchform 6 dar.

Die gemittelte Rauhtiefe auf der Oberfläche 12 der - nach einer neuerlichen kurzen Trocknung durch das Eintauchen der Tauchform 6 die Trägerschicht 3 aufgebrachten-Gleitschicht 9 wird dabei in gleicher Art durch die Einzelrauhtiefen 26 bis 30 in fünf aneinandergrenzenden Einzelmeßstrecken mit einer gleichen Länge 31 ermittelt.

Die Gestaltsabweichungen 19 und 20 in dieser Oberfläche 12 werden aber nun durch die Oberflächenmorphologie der Tauchform 6 entweder überhaupt nicht beeinflußt oder in entsprechend den gewählten Verfahren verringertem Ausmaß, sondern vielmehr durch das Herstellungsverfahren beim Herstellen der Gleitschicht 9 gebildet.

In der Stirnansicht des Handschuhs 4 in Fig. 3 ist ersichtlich, daß die gesamte die Innenseite des Handschuhs 4 bildende Oberfläche 12 mit entsprechenden Gestaltsabweichungen 19, 20, entsprechend der vorstehenden Beschreibung, versehen ist.

Dem gegenüber ist beispielsweise die die Außenseite des Handschuhs 4 bildende Unterseite 11 nur in jenem Bereich, der oberhalb der Handinnenfläche zu liegen kommt, mit entsprechenden Gestaltsabweichungen 14, d.h. mit einer Aufrauhung versehen, um beispielsweise die Griffsicherheit vor allem beim Einsatz der Handschuhe im medizinischen Bereich zum Erfassen der Werkzeuge oder auch im Montagebereich zu verbessern. Selbstverständlich ist es aber auch möglich, daß die gesamte Unterseite 11, also die gesamte Außenfläche des Handschuhs 4, mit entsprechenden Gestaltsabweichungen 14 und mit einer dadurch gebildeten Rauhung ausgestattet ist. In welchem Ausmaß der Teilbereich bzw. eine Teilfläche des Teilbereiches 18 mit Gestaltsabweichungen 14 bzw. einer Aufrauhung versehen ist, kann durch die Ausgestaltung der Tauchform 6, insbesondere deren Oberflächenrauhigkeit, festgelegt werden.

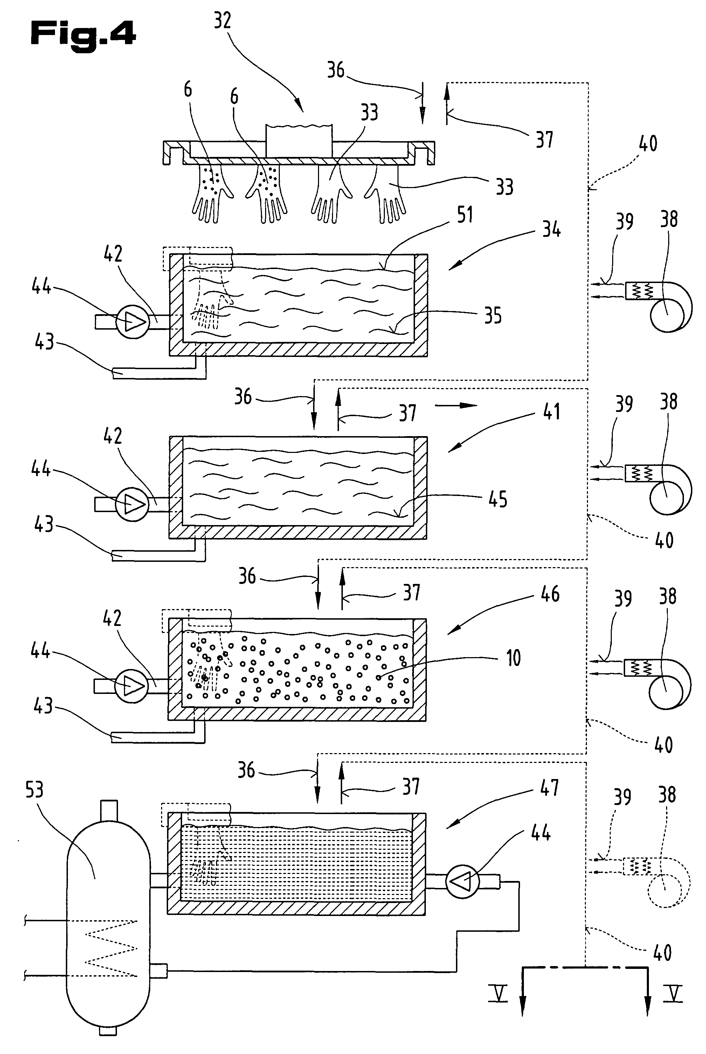

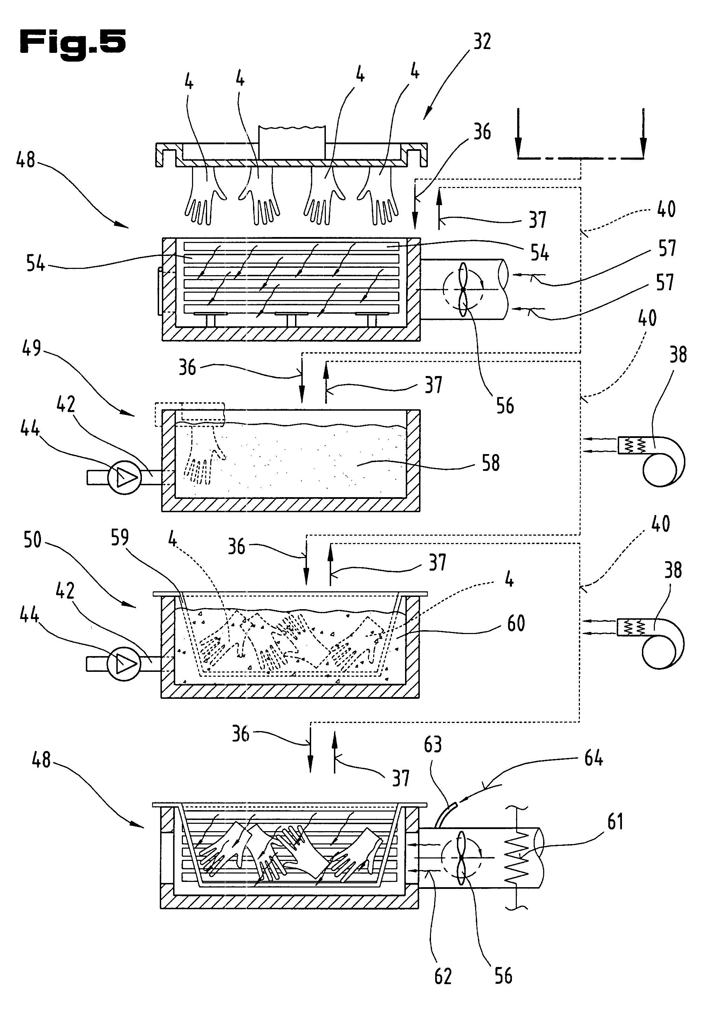

Der besseren Übersichtlichkeit wegen soll dieses Herstellungsverfahren, welches zu der in den Zeichnungen Fig. 2 und 3 schematisch und völlig unproportional dargestellten gemittelten Rauhtiefe führt, anhand des schematisch durch einzelne, aufeinander folgende Vorrichtungen und Vorrichtungsteile in den Fig. 4 und 5 dargestellten Verfahrensablaufes zum Herstellen eines derartigen Gegenstandes 1 bzw. Handschuhs 4 im einzelnen erläutert werden.

Zu der Herstellung von bevorzugt puderfreien Handschuhen 4 aus einem Latex-Produkt 2 sind auf einem Formträger 32 mehrere Tauchformen 6, wie z.B. aus Porzellan, Kunststoff, Metall, Aluminium oder dgl., mit einer die gewünschten Gestaltsabweichungen 14 aufweisenden Oberfläche 13 gemäß der vorangegebenen gemittelten Rauhtiefe versehen, während Tauchformen 33 mit einer glatten Oberfläche für die spätere Außenseite eines herzustellenden Handschuhs 4 aus Latex angeordnet sind.

Dem Formträger 32 ist ein Tauchbecken 34 zugeordnet, in dem sich eine wäßrige Koagulationslösung 35 befindet. Diese kann, wie bereits einleitend angegeben, Kalziumnitrat, Polyethylenglykol und einen wasserlöslichen Silikontyp oder Kreide enthalten. Wie mit dem Pfeil 36 angedeutet, wird der Formträger 32 in das Tauchbecken 34 eingetaucht, dann gemäß Pfeil 37 aus diesem herausgenommen und gegebenenfalls nach einer Zwischentrocknung - wie symbolisch durch ein Heißluftgebläse 38 und die die erhitze Luft zum Trocknen schematisch darstellenden Pfeile 39 angedeutet - entsprechend den punktierten Linien 40 zu einem weiteren Tauchbecken 41 verbracht.

Selbstverständlich ist es im Rahmen dieses erfindungsgemäßen Verfahrens dem Fachmann überlassen, ob das Eintauchen der Tauchformen 6 bzw. des Formträgers 32 immer in dasselbe Tauchbecken 34 erfolgt und dieses über einen Zulauf 42 und einen Ablauf 43, z.B. über eine schematisch dargestellte Pumpe 44, abwechselnd mit der Koagulationslösung 35 oder dem Latex-Produkt 45 oder beliebig anderen Flüssigkeiten oder Medien gefüllt wird oder ob mehrere hintereinander angeordnete Tauchbecken 34, 41, 46, 47, 48, 49, 50 vorgesehen sind. Auch ist es möglich, daß das Eintauchen der Tauchformen 6 durch eine Relativbewegung beliebiger Art zwischen dem Formträger 32 und dem Tauchbecken 34, 41 und 46 bis 50 erfolgt. Selbstverständlich ist es auch möglich, daß vor allem jene Behandlungs- oder Verfahrensschritte, bei welchen die Gegenstände 1 bzw. Handschuhe 4 bereits von den Tauchformen 6, 33 entfernt sind, in rotierenden Behältern, beispielsweise Trommeln oder dgl., wie sie auch bei herkömmlichen Wasch- und Trockenmaschinen verwendet werden, erfolgen, um sie während der Behandlung durch ständige Rotation der Trommel zu bewegen, um beispielsweise auch ein Aneinanderkleben der Gegenstände 1 bzw. Handschuhe 4 während der Produktion zu verhindern.

Da alle diese unterschiedlichen Möglichkeiten für die Durchführung des Verfahrens dem Fachmann aus dem breiten Stand der Technik bekannt sind, wird auf die Darstellung der unterschiedlichen Vorrichtungen zur Durchführung der einzelnen Verfahrensschritte verzichtet und diese nur anhand der rein schematischen Darstellungen in den Fig. 4 und 5 erläutert.

Nach dieser gegebenenfalls erfolgten Zwischentrocknung des Koagulanten bzw. der Koagulationslösung 35 werden die Tauchformen 6 in das Tauchbecken 41 eingetaucht, sodaß die Tauchformen 6 und 33 zur Gänze unter einen Flüssigkeitsspiegel 51 eines Latex-Produktes 45 beliebiger Zusammensetzung, gemäß den zuvor getätigten Angaben, eintauchen. Die Tauchformen 6 verbleiben solange in dem Tauchbecken 41, bis sich eine gewünschte Schichtdicke von flüssigem Latex-Produkt 45 auf der Oberfläche der Tauchformen 6 bzw. der Schicht aus der Koagulationslösung 35 anlagern kann. Dazu ist es auch möglich, daß die Tauchformen 6, 33 mehrmals aus dem Tauchbecken 41 herausgezogen und wieder eingetaucht werden.

Danach wird entsprechend der punktierten Linie 40 eine kurze Zwischentrocknung des Latex-Produktes 45 auf den Tauchformen 6, 33 beispielsweise mit Heißluft an dem weiteren Heißluftgebläse 38 vorgenommen, wozu die Luft eine Temperatur von 70°C bis 140°C, bevorzugt 90°C bis 110°C, aufweisen kann und die Tauchformen 6 dieser Heißluft 15 sec. bis 60 sec. ausgesetzt werden können. Bevorzugt wird die Dauer der Heißluftbeaufschlagung und die Temperatur der Heißluft so gesteuert, daß die Schicht aus dem flüssigen Latex-Produkt 45 an ihrer Oberfläche vom flüssigen in den gelartigen oder festen Zustand übergeht.

Daraufhin wird der Formträger 32 mit den Tauchformen 6, 33 in das Tauchbecken 46 gemäß den Pfeilen 36, 37 eingetaucht, in dem sich ein polymeres Material 10 zur Herstellung der Gleitschicht 10 befindet. An dieser Stelle sei der Ordnung halber erwähnt, daß es selbstverständlich möglich ist, das polymere Material 10 bzw. natürlich auch das Latex-Produkt 45 bzw. die Koagulationslösung 35 durch Zusatz eines oder mehrerer dem Fachmann aus dem bekannten Stand der Technik in unterschiedlichen Ausführungen bekannten Viskositätsregler in Ihrer Viskosität an die gewünschten Eigenschaften anzupassen. Dadurch ist es möglich, die Viskosität der üblicher Weise als Dispersionen vorliegenden Produkte bzw. Materialien auf die jeweils gewünschten Einsatzzwecke anzupassen, um entsprechende Schichtstärken beim Tauchen oder eine gleichmäßige Ablagerung der verschiedenen Materialien an einer Oberfläche der Tauchformen 6, 33 zu erreichen. Der Ordnung halber sei an dieser Stelle auch erwähnt, daß in der vorliegenden Beschreibung immer wieder der Begriff "Latex" durchgehend verwendet wurde, unabhängig davon, ob es sich um eine Dispersion oder die ausgehärtete, trockene, vernetzte Schicht oder die feste Phase dieses Materials handelt, auch wenn diese wie z.B. bei Naturlatex üblicherweise als Kautschuk bezeichnet wird. Es wäre daher auch möglich, die aus den einzelnen vorgenannten Materialien gebildeten Schichten als Kautschukschichten zu bezeichnen. Dieses polymere Material 10 kann entsprechend den vorstehenden Angaben zusammengesetzt sein.

Nach dem Herausheben des Formträgers 32 mit den Tauchformen 6, 33 aus der Dispersion aus polymerem Material zur Herstellung der Gleitschicht 9, wird diese zusätzlich aufgebrachte Gleitschicht 9, wie schematisch angedeutet, mit Heißluft beispielsweise bei einer Temperatur zwischen 70°C und 140°C, bevorzugt 90°C bis 110°C, über eine Dauer von 15 sec. bis 60 sec. angetrocknet. Bevorzugt wird die Temperatur und die Zeitdauer der Heißluftbehandlung so abgestimmt, daß die Oberfläche der Gleitschicht 9 in einen gelartigen bzw. festen Zustand übergeht.

Unmittelbar darauf werden die Tauchformen 6, 33 mit den darauf befindlichen Rohteilen der Gegenstände 1 bzw. Handschuhe 4 in ein weiteres Tauchbecken 47 eingetaucht, in welchem die Gleitschicht 9 mit heißem Wasser 52 besprüht oder gespült wird. Dazu kann das heiße Wasser 52 mittels einer Pumpe 44 von einem Heizkessel bzw. einem Wärmetauscher 53, durch die es einer gewünschten Temperatur zwischen 40°C und 95°C, bevorzugt 70°C bis 90°C, gehalten wird, im Kreislauf durch das Tauchbecken 47 hindurchgefördert werden.

Durch diese Behandlung mit heißem Wasser 52 wird die chemische Reaktion in der Gleitschicht 9 und der Trägerschicht 3 eingeleitet bzw. unterstützt, sodaß es zu einem Beginn oder zu einer vollständigen Koagulation in dieser Schicht kommt.

Nach dieser Heißwasserbehandlung, die zwischen 15 sec. und 8 min, bevorzugt 30 sec. und 4 min, andauern kann, werden die Gegenstände 1 bzw. die Handschuhe 4 in ihrer auf den Tauchformen 6, 33 aufgezogenen Stellung aus dem Tauchbecken 47 entfernt, sodaß das restliche Wasser 52 abtropfen kann.

Die Fortsetzung des Verfahrensablaufes ist gemäß der Schnittlinie V - V in Fig. in Fig. 5 gezeigt. Die aus dem Wasser 52 entfernten Handschuhe 4 werden über einen Zeitraum von 10 sec. bis 180 sec., bevorzugt 20 sec. bis 50 sec., die als Entwässerungszeit dient, im Freien belassen bzw. einer Vortrocknung mittels Heißluft ausgesetzt, bevor sie in das Tauchbecken, in diesem Fall einem Wärmeschrank bzw. einem Wärmeofen, mittels einer Infrarotstrahlung beaufschlagt werden, sodaß die Trägerschicht 3 und die Gleitschicht 9 endgültig fixiert werden. Die bevorzugt als Kachelflächen ausgebildeten Abstrahlungsflächen 54 für die Infrarotstrahlung weisen eine Temperatur zwischen 350°C und 700°C auf und werden die Gegenstände 1 bzw. Handschuhe 4 über eine Zeitdauer zwischen 1 min und 5 min dieser schematisch durch Pfeile 55 angedeuteten Infrarotstrahlung ausgesetzt. Dazu ist es nötig, daß durch das Tauchbecken 48 bzw. eine entsprechende Trockenvorrichtung mittels eines Ventilators 56 die Umgebungsluft gemäß den schematisch angedeuteten Pfeilen 57 durch die Wärmekammer bzw. den Trockner oder das Tauchbecken 48 hindurchgeblasen werden kann.

Nach der Trocknung der Gegenstände 1 bzw. Handschuhe 4 werden, wie in Fig. 5 gezeigt, die Tauchformen 6, 33 mit ihren Formträgern 32 in das Tauchbecken 49 eingetaucht, in dem sich ein Trennmittel 58, beispielsweise ein Puder wie Talkum, Kreide oder Maisstärkepulver oder eine Silikonemulsion befindet, welches beim nachfolgenden Abstreifen und gleichzeitigem Wenden der Gegenstände 1 bzw. Handschuhe 4 von den Tauchformen 6, 33 ein Zusammenkleben der den Tauchbecken 34, 41 und 46 bis 48 zugewandten Oberfläche 12 verhindern soll.

Nach dem Abstreifen werden die Gegenstände 1 bzw. die Rohteile der Handschuhe 4 beispielsweise in einen Transportkorb 59, der auch gitterförmig oder mit Durchbrüchen versehen sein kann, eingefüllt und wird dieser Transportkorb 59 in das Tauchbecken 50 eingebracht, in dem sich ein Bleichmittel 60 in einer aus dem Stand der Technik bekannten Zusammensetzung befindet. So kann beispielsweise 0,5%iges Natrimhypochlorid mit ca. 0,2 % Salzsäure vermischt verwendet werden.

Anschließend an das Behandeln mit dem Bleichmittel 60 werden die Gegenstände 1 bzw. Handschuhe 4 zumindest einmal, bevorzugt jedoch mehrmals, in Wasser oder aus dem Stand der Technik bekannten Flüssigkeiten gewaschen, um die Reste an Bleichmitteln oder Säuren von z.B. getrennte Behandlungen mit Säuren und Bleichmittel zu entfernen.

Wenn dieser Waschvorgang abgeschlossen ist, werden die Gegenstände 1 bzw. Handschuhe mit dem Transportkorb 59 in ein Tauchbecken 48 bzw. in eine Trockenkammer verbracht, in der entweder durch in der Trockenkammer oder aber auch in einem Ansaugluftweg der Luft zum Lufttrocknen Heizkörper 61 angeordnet sein können, sodaß die Trocknung mittels der durch Pfeile 62 schematisch angedeuteten, Luft, die über den Ventilator 56 oder ein Gebläse durch den Innenraum der Trockenkammer hindurchgeführt werden kann, erfolgt.

Selbstverständlich ist auch jede andere Form der Trocknung möglich. So auch beispielsweise unter Verwendung von Trommeln oder dgl.. Bevorzugt ist es bei Verwendung einer derartigen Trockenkammer möglich, über eine zusätzliche Leitung 63 eine Silikonemulsion in den Luftstrom gemäß dem dargestellten Pfeil 64 einzublasen, um gegen Ende des Trocknungsvorganges oder am Schluß des Trocknungsvorganges, beispielsweise die die Außenseiten und gegebenenfalls die Innenseiten des Handschuhs 4 bildenden Oberflächen mit Silikon zu beschichten. Selbstverständlich ist es aber auch möglich, diese Silkonbeschichtung in ebenso bekannter Weise durch Tauchverfahren in Emulsionen oder durch Spritzauftrag in bestimmten Teilbereichen der Gleitzonen bzw. der Oberfläche 12 oder 13 aufzubringen.

Bei dem vorstehend beschriebenen Verfahren wird die erfindungsgemäße Rauheit bzw. die gemittelte Rauhtiefe in der Gleitschicht 9 dadurch erreicht, daß während bzw. unmittelbar nach der Beaufschlagung der Gleitschicht 9 mit heißem Wasser 52 der Gleitschicht 9 zwischen 40 % bis 70 %, bevorzugt 50 % bis 60 % des Wassergehaltes entzogen werden. In Folge des raschen Wasserentzuges kommt es zum Aufbau relativ hoher Oberflächenspannungen im Bereich der Gleitschicht 9 und führt dies zu einem Einsinken bzw. einer Verringerung der wie in Fig. 1 gezeigten Dicke der Gleitschicht 9, die zwischen 2 µm und 80 µm, bevorzugt 2 µm bis 30 µm, betragen kann und beispielsweise der Tiefe 24 in Fig. 1 entspricht, bei welcher die Gestaltsabweichung 19 so groß ist, daß sie sich über die gesamte Dicke der Gleitschicht 9, d.h. bis auf die Oberseite 8 der Trägerschicht 3 erstreckt.

Diese Bereiche mit verringerter Wandstärke der Gleitschicht 9 bilden die Gestaltsabweichungen 19, 20, die zum überwiegenden Teil von der netzartigen Struktur 23 bzw. deren Stegen 21, 22 umgrenzt sind. Durch diese erhabenen Stege 21, 22 der netzartigen Struktur 23 liegt der Gegenstand 1 bzw. der Handschuh 4 nur entlang der Stege auf der Oberfläche der menschlichen Haut auf und wird zum einen die Berührungs-fläche mit der Haut wesentlich verringert, zum anderen dadurch die Schlüpfrigkeit, insbesondere die Naßschlüpfrigkeit erheblich erhöht. Gleichzeitig werden dadurch Hohlräume geschaffen, die auch den beim Arbeiten entstehenden Schweiß aufnehmen können, sodaß auch der Tragekomfort derartiger Gegenstände 1 bzw. Handschuhe 4 erheblich verbessert wird.

Dieser verbesserte Gleiteffekt kann durch das zusätzliche Aufbringen einer Silikonschicht, beispielsweise durch Eintauchen in eine Silikonemulsion oder durch Beisetzen einer Silikonemulsion zur Trocknungsluft noch zusätzlich verstärkt werden.

Werden die Gegenstände 1 bzw. Handschuhe 4 in loser Form einen Bleichvorgang zum Bleichen der vom Tauchbad beim Auftragen der Gleitschicht 9 abgewandten Seite, die dann die Außenseite des Gegenstandes 1 bzw. Handschuhs 4 bildet, zugeführt, so kann dadurch auch die Schlüpfrigkeit des Gegenstandes 1 bzw. Handschuhs 4 zusätzlich noch verbessert werden.

Nach dem durchgeführten Trocknungsvorgang im Tauchbecken 50 werden die Handschuhe 4 dann paarweise zusammensortiert und den weiteren Bearbeitungsvorgängen, wie Sterilisation und Verpackung, zugeführt.

Die erfindungsgemäße Rauheit kann aber auch durch viele andere bekannte Verfahren verstärkt werden.

Die einfachste Methode zur Aufrauhung von Gegenständen 1 zusätzlich zu der produktionsbedingten Rauhtiefe liegt darin, die mit dem noch weichen Gegenstand 1 bzw. der Gleitschicht 9 belegte Tauchform 6, 33 beispielsweise durch eine Sandstrahlkammer oder durch ein Fließbett mit feinen Glasperlen zuführen. Überschüssige Partikel aus dem Strahlverfahren müssen üblicherweise in nachfolgenden Prozeßschritten vollständig entfernt werden.

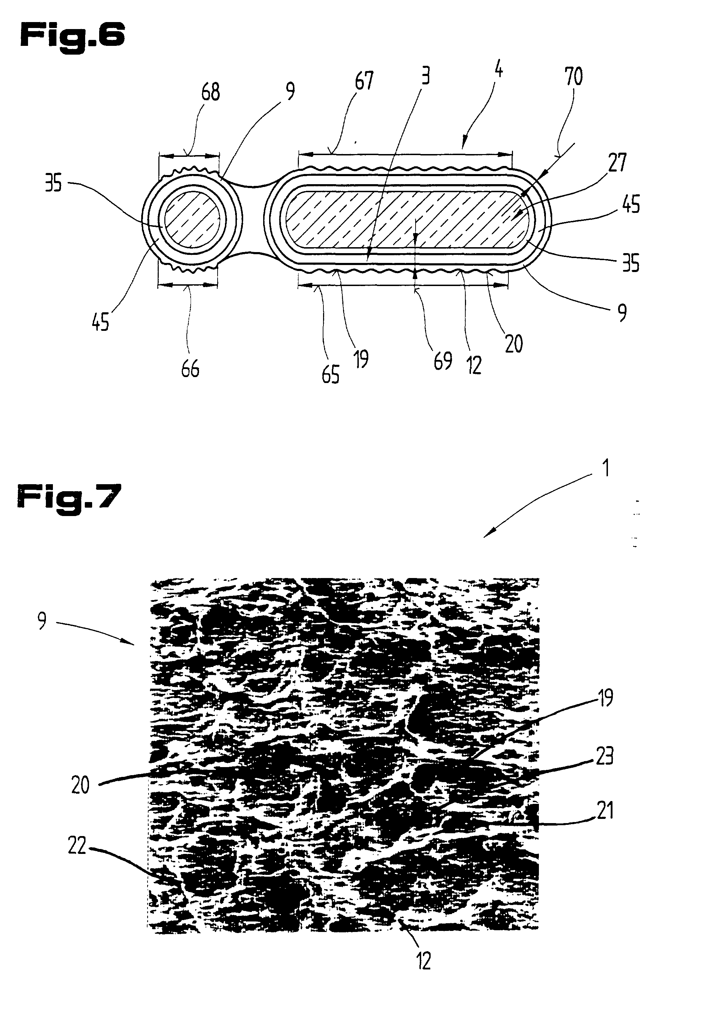

Schließlich ist in Fig. 6 anhand eines Schnittes durch einen auf einer Tauchform 33 angeordneten Handschuh 4 gezeigt, daß auf der beispielsweise glatt ausgebildeten Oberfläche im Tauchvorgang zuerst eine Koagulationslösung 35 und dann ein Latex-Produkt 45 aufgebracht wird, worauf die Gleitschicht 9 aufgetaucht bzw. aufgesprüht wird.

Durch eine entsprechende Ausbildung der Einrichtungen zum Einprägen der gemittelten Rauhtiefe bzw. der Gestaltsabweichung 19, 20 in der Oberfläche 12 können beispielsweise über die zuvor angesprochenen Glasperlen oder entsprechende Preßstempel vor der Herstellung der zuvor beschriebenen Aufrauhungen aufgrund der Gestaltsabweichungen 19, 20 zusätzlich in den Teilflächen 65 bis 68 aufgerauht werden.

Diese Teilflächen erstrecken sich beispielsweise nur über einen Teil der horizontalen Anlageflächen des Gegenstandes 1 an der Hand des Benutzers. Damit wird, ohne daß der gesamte Artikel zu stark geschwächt wird eine ausreichende Naßschlüpfrigkeit erreicht, da vielfach nur auf jenen Flächen, auf die eine Vorspannung zum festen Sitz des Gegenstandes 1 auf einen Körperteil des Benutzers einwirkt, eine hohe Gleitfähigkeit erzielt werden muß. Im Rahmen der Erfindung ist es natürlich auch möglich, daß durch entsprechende Vorkehrungen beispielsweise partiellen Aufbringen von Gleitmitteln oder dgl. vor dem Aufbringen der Gleitschicht 9 auch die Gleitschicht 9 oder zumindest die Gestaltsabweichungen 19, 20 nur auf einer oder einzelnen der Teilflächen 65 bis 68 hergestellt werden.

Aus der Darstellung in Fig. 6 ist weiters ersichtlich, daß die sich aus der Koagulationslösung 35 und dem Latex-Produkt 45 gebildete Trägerschicht 3 eine Dicke 69 zwischen 100 µm und 300 µm aufweisen kann.

Die auf dieser Trägerschicht 3 angeordnete Gleitschicht 9 weist dagegen beispielsweise eine Dicke 70 von 2 µm bis 80 µm, bevorzugt 2 µm bis 30 µm, auf.

In Fig. 7 ist eine Draufsicht auf einen Teil der Oberfläche 12 der Gleitschicht 9 eines Gegenstandes 1 gezeigt. Aus dieser Darstellung ist sehr gut die netzartige Struktur 23, die durch die Stege 21, 22 gebildet wird und die Gestaltsabweichungen 19, 20 umgrenzen, ersichtlich.

Bei dieser Darstellung handelt es sich um eine mit einem Elektronenmikroskop hergestellte 100-fache Vergrößerung der Oberfläche 12 der Gleitschicht 9.

Die in den vorhergehenden Figuren beschriebenen Varianten der Materialauswahl für die Trägerschicht 3 und die Gleitschichten 9 sind wahlweise einsetzbar. Selbstverständlich können die einzelnen vorstehend beschriebenen Ausführungsbeispiele und die in diesen Ausführungsbeispielen gezeigten Varianten und unterschiedlichen Ausführungen jeweils für sich eigenständige, erfinderische Lösungen bilden und beliebig miteinander kombiniert werden. Vor allem können die einzelnen in den Fig. 1, 2, 3, 4, 5; 6; 7; gekennzeichneten Ausführungen den Gegenstand von eigenständigen, erfindungsgemäßen Lösungen bilden. Die diesbezüglichen erfindungsgemäßen Aufgaben und Lösungen sind den Detailbeschreibungen dieser Figuren zu entnehmen.

Der Ordnung halber sei abschließend darauf hingewiesen, daß zum besseren Verständnis der Erfindung die einzelnen Ausführungsbeispiele teilweise unmaßstäblich verzerrt bzw. vergrößert dargestellt wurden. Es können auch einzelne Merkmale der in den einzelnen Ausführungsbeispielen gezeigten Merkmalskombinationen jeweils für sich eigenständige, erfindungsgemäße Lösungen bilden.

## Patentansprüche

1. Gegenstand (1) aus einer Trägerschicht (3) aus einem flexiblen Gummi und/oder Kunststoff, der zumindest mit einem Teilbereich (7) der Oberfläche zur Berührung oder zur Anlage mit einer menschlichen Haut ausgebildet ist, insbesondere ein Handschuh (4), ein Kondom oder dgl., der in einem Teilbereich (7, 18) der Oberfläche mit einer Gleitschicht (9) aus polymerem Material (10) versehen ist, dadurch gekennzeichnet, daß zumindest eine Teilfläche (65 bis 68) der Oberfläche (12) als Vertiefungen und/oder Erhebungen aus dem polymeren Material (10) gebildet ist, wobei die Gleitschicht (9) auf der Trägerschicht (3) fixiert ist, und das Verhältnis der mittleren Abstände (15, 16) zwischen den Erhebungen bzw. den gegenüber einer erhabenen netzartigen Struktur (23) wiederkehrenden Vertiefungen zu ihrer Tiefe (17; 24, 25) zwischen 500 : 1 und 5 : 1 liegt.

2. Gegenstand nach Anspruch 1, dadurch gekennzeichnet, daß zwischen 50 % und 90 %, bevorzugt 60 % bis 80 %, der Teilfläche (65 bis 68) vertieft ausgebildet sind.

3. Gegenstand nach Anspruch 1 oder 2, dadurch gekennzeichnet, daß zumindest 50 % der Vertiefungen und/oder Erhebungen in der Draufsicht auf die Gleitschicht (9) allseitig von der netzartigen Struktur (23) umgrenzt sind.

4. Gegenstand nach einem oder mehreren der vorhergehenden Ansprüche, dadurch gekennzeichnet, daß das Material (10) der Gleitschicht (9) durch zumindest einen Natur- und/oder Syntheselatex gebildet ist.

5. Gegenstand nach einem oder mehreren der vorhergehenden Ansprüche, dadurch gekennzeichnet, daß das Material (10) ein Polyurethan und/oder Polyacrylate und/oder Polymetacrylate und/oder Polysiloxane oder vergleichbare Produkte enthält.

6. Gegenstand nach Anspruch 5, dadurch gekennzeichnet, daß die Gleitschicht (9) 0 Gew% bis 30 Gew% Polyurethan, 1 Gew% bis 40 Gew% Polyacrylat, 1 Gew% bis 20 Gew% Polysiloxan, 0 Gew% bis 40 Gew% Füllstoffe enthält.

7. Gegenstand nach Anspruch 6, dadurch gekennzeichnet, daß der Füllstoff pulver- oder puderförmig durch Materialien wie Kreide, Kalk, Maisstärke, Siliziumdioxyd und/oder Gips gebildet ist.

8. Gegenstand nach einem oder mehreren der vorhergehenden Ansprüche, dadurch gekennzeichnet, daß das polymere Material (10) stark hydrophile Acryl- und/oder Metacrylsäure- und/oder Acrylat- und/oder Metacrylat-Gruppen enthält.

9. Gegenstand nach einem oder mehreren der vorhergehenden Ansprüche, dadurch gekennzeichnet, daß das Material (10) eine hohe Oberflächenadhäsion aufweist und bevorzugt unvernetzt ist.

10. Gegenstand nach einem oder mehreren der vorhergehenden Ansprüche, dadurch gekennzeichnet, daß zumindest auf die Bereiche der Gleitschicht (9), welche mit einer Aufrauhung versehen sind, ein Silikon aufgebracht ist.

11. Gegenstand nach Anspruch 10, dadurch gekennzeichnet, daß die Menge des Silikons zumindest auf der Oberfläche der Teilbereiche (7, 18) zwischen 0,05 g/m² bis 3 g/m², bevorzugt 0,25 g/m², beträgt.

12. Gegenstand nach einem oder mehreren der vorhergehenden Ansprüche, dadurch gekennzeichnet, daß die Gleitschicht bzw. -schichten (9) auf der inneren Oberfläche (12) der hüllenartigen Trägerschicht (3) aufgebracht sind.

13. Gegenstand nach einem oder mehreren der vorhergehenden Ansprüche, dadurch gekennzeichnet, daß die Gleitschicht bzw. -schichten (9) auf der äußeren Oberfläche (12) der hüllenartigen Trägerschicht (3) aufgebracht ist bzw. sind.

14. Gegenstand nach einem oder mehreren der vorhergehenden Ansprüche, dadurch gekennzeichnet, daß die gemittelte Rauhtiefe in Teilbereichen mit geringerer Relativbewegung zwischen dem Gegenstand (1) und der Haut eines Benutzers geringer ist.

15. Gegenstand nach einem oder mehreren der vorhergehenden Ansprüche, dadurch gekennzeichnet, daß zumindest eine Lage der Trägerschicht (3) aus einem Latex-Produkt (2) gebildet ist, welches ein Polymer (5), z.B. ein Natur- und/oder Synthese-Latex, enthält.

16. Gegenstand nach einem oder mehreren der vorhergehenden Ansprüche, dadurch gekennzeichnet, daß die Gleitschicht (9) auf einer aus zumindest einer Lage bestehenden Trägerschicht (3) angeordnet ist, die leicht vernetzt ist.

17. Gegenstand nach Anspruch 15, dadurch gekennzeichnet, daß das Latex-Produkt (2) vorvernetzt ist.

18. Gegenstand nach einem oder mehreren der vorhergehenden Ansprüche, dadurch gekennzeichnet, daß die Trägerschicht (3) durch eine wäßrige Lösung eines Koagulant, die Kalziumnitrat, Polyethylenglycol und ein wasserlösliches Silikon oder Kreide enthält, und eine vorvernetzte Naturlatexmischung gebildet ist.

19. Gegenstand nach einem oder mehreren der vorhergehenden Ansprüche, dadurch gekennzeichnet, daß die Trägerschicht (3) einen insbesondere vorvernetzten Syntheselatex, z.B. Chloroprenlatex, NBR-Latex, X-NBR-Latex oder SBR-Latex, aufweist.

20. Gegenstand nach einem oder mehreren der vorhergehenden Ansprüche, dadurch gekennzeichnet, daß die Trägerschicht (3) an ihrer Oberfläche chloriert ist.

21. Gegenstand nach Anspruch 20, dadurch gekennzeichnet, daß die durch die Chlorierung aufgeworfenen, erhöhten Randbereiche der Oberflächenverformungen über die benachbarten Oberflächenzonen um eine Höhe von 0,5 um bis 10 µm, bevorzugt ≤ 1 µm, vorragen.

22. Gegenstand nach einem oder mehreren der vorhergehenden Ansprüche, dadurch gekennzeichnet, daß dieser als Gummihandschuh ausgebildet ist.

23. Gegenstand nach einem oder mehreren der vorhergehenden Ansprüche, dadurch gekennzeichnet, daß dieser als medizinischer Handschuh ausgebildet ist.

24. Gegenstand nach einem oder mehreren der vorhergehenden Ansprüche dadurch gekennzeichnet, daß die gemittelte Rauhtiefe zwischen 0,5 µm und 100 µm, bevorzugt zwischen 1 µm und 40 µm beträgt.

25. Verfahren zum Herstellen eines flexiblen Gegenstandes (1), beispielsweise eines Handschuhs (4), eines Kondoms, bei dem eine Trägerschicht (3) aus Kunststoff und/ oder Gummi hergestellt wird, dadurch gekennzeichnet, daß auf diese in zumindest einem Teilbereich (7, 18) ihrer Oberfläche eine Gleitschicht (9) aufgetaucht wird, die unmittelbar anschließend 10 sec. bis 600 sec. angetrocknet wird, worauf die Gleitschicht (9) mit Wasser mit einer Temperatur zwischen 40°C und 100°C behandelt oder mit dem heißen Wasser gespült wird, sodaß sich danach der Wassergehalt der Gleitschicht (9) um ca. 40 % bis 70 % verringert, und bei dem im Bereich der von der Trägerschicht (3) abgewandten Oberfläche (12) der Gleitschicht (9) eine Rauheit mit Vertiefungen entsteht, wobei das Verhältnis von mittleren Abständen (15, 16) zwischen den gegenüber einer erhabenen netzartigen Struktur (23) wiederkehrenden Vertiefungen zu ihrer Tiefe (24, 25) zwischen 500:1 und 5:1 liegt.

26. Verfahren nach Anspruch 25, dadurch gekennzeichnet, daß die Antrocknung mit Heißluft erfolgt.

27. Verfahren nach Anspruch 25 oder 26, dadurch gekennzeichnet, daß die Wassertemperatur zwischen 60 °C und 80 "C beträgt.

28. Verfahren nach einem der Ansprüche 25 bis 27, dadurch gekennzeichnet, daß der Wassergehalt um 50 % bis 60 % verringert wird.

29. Verfahren nach einem der Ansprüche 25 bis 28, dadurch gekennzeichnet, daß unmittelbar anschließend an die Entwässerungszeit nach dem Spülen des Gegenstandes (1) mit dem heißen Wasser der Gegenstand (1) einer Infrarotstrahlung ausgesetzt wird.

30. Verfahren nach Anspruch 29, dadurch gekennzeichnet, daß Strahlungskörper zur Abgabe der Infrarotstrahlung, z.B. Kacheln, eine Temperatur zwischen 350°C und 700°C aufweist.

31. Verfahren nach einem der Ansprüche 29 oder 30, dadurch gekennzeichnet, daß die Dauer der Infrarotbestrahlung zwischen 1 und 5 Minuten beträgt.

32. Verfahren nach einem der Ansprüche 25 bis 31, dadurch gekennzeichnet, daß die Entwässerungszeit zwischen dem Spülen des Gegenstandes (1) mit dem heißen Wasser und der Einwirkung der Infrarotstrahlung zwischen 10 und 180 Sekunden, bevorzugt zwischen 20 und 50 Sekunden, beträgt.

33. Verfahren nach einem der Ansprüche 25 bis 32, dadurch gekennzeichnet, daß der Gegenstand (1) während der Entwässerungszeit mit Heißluft beaufschlagt wird.

34. Verfahren nach einem der Ansprüche 26 bis 33, dadurch gekennzeichnet, daß beim mit Heißluft durchgeführten Antrocknen der Koagulationslösung des Latex-Produktes (2) und/oder der Gleitschicht (9) diese an ihrer Oberfläche vom flüssigen in den gelförmigen bzw. festen Zustand überführt wird.

35. Verfahren nach Anspruch 34, dadurch gekennzeichnet, daß das Antrocknen der Oberfläche des Latex-Produktes (2) und/oder der Gleitschicht (9) bei einer Temperatur von 70°C bis 140°C, bevorzugt 90°C bis 110°C, erfolgt.

36. Verfahren nach einem der Ansprüche 34 oder 35, dadurch gekennzeichnet, daß der Antrockenvorgang des Latex-Produktes (2) und der Gleitschicht (9) zwischen 10 und 80 Sekunden, bevorzugt 15 bis 60 Sekunden, beträgt.

37. Verfahren nach einem der Ansprüche 25 bis 36, dadurch gekennzeichnet, daß die Temperatur des heißen Wassers zum Spülen der Gleitschicht (9) zwischen 40°C und 95°C, bevorzugt 70°C bis 90°C und die Behandlungsdauer zwischen 15 Sekunden bis 8 Minuten, bevorzugt 30 Sekunden bis 4 Minuten beträgt.

38. Verfahren nach einem der Ansprüche 25 bis 37, dadurch gekennzeichnet, daß die Gleitschicht (9) aus einer Dispersion aus zumindest einem polymeren Material (10) gebildet ist.

39. Verfahren nach Anspruch 38, dadurch gekennzeichnet, daß die Dispersion durch ein Gemisch aus 0 Gew% bis 30 Gew% Polyurethan-, 1 Gew% bis 40 Gew% Polyacrylat-, 1 Gew% bis 20 Gew% Polysiloxandispersion, 0 Gew% bis 10 Gew% Füllstoffe und dem restlichen Anteil aus Wasser gebildet ist.

40. Verfahren nach Anspruch 39, dadurch gekennzeichnet, daß der Füllstoff pulver- oder puderförmig durch Materialien wie Kreide, Kalk, Maisstärke, Siliziumdioxyd und/oder Gips gebildet ist.

41. Verfahren nach einem der Ansprüche 38 bis 40, dadurch gekennzeichnet, daß die Viskosität der Dispersion der Gleitschicht (9) zwischen 5 mPa.s bis 30 mPa.s, bevorzugt 7 mPa.s bis 11 mPa.s, beträgt.

42. Verfahren nach einem der Ansprüche 25 bis 41, dadurch gekennzeichnet, daß die Rauheit durch die Formflächen an der Trägerschicht (3) ausgeformt wird.

43. Verfahren nach einem der Ansprüche 25 bis 42, dadurch gekennzeichnet, daß die Vertiefungen durch das Einpressen von Mikrokugeln oder durch Sandstrahlung hergestellt werden.

44. Verfahren nach einem der Ansprüche 25 bis 43, dadurch gekennzeichnet, daß auf die gerauhten Oberflächen der Teilbereiche ein Gleitmittel, insbesondere ein Silikon, aufgetragen wird.

45. Verfahren nach einem der Ansprüche 25 bis 44 dadurch gekennzeichnet, daß die gemittelte Rauhtiefe zwischen 0,5 µm und 100 µm liegt.

## Claims

1. Article (1) made from a base layer (3) of a flexible rubber and/or synthetic material, at least a part region (7) of the surface of which is intended to come into contact or lie against human skin, in particular a glove (4), a condom or similar, a part region (7, 18) of the surface being provided with an anti-friction layer (9) of polymer material (10), characterised in that at least a part surface (65 to 68) of the surface (12) has recesses and/or raised portions in the polymer material (10), the anti-friction layer (9) being fixed to the base layer (3) and the ratio of the mean distances (15, 16) between the raised portions or recurring recesses relative to a raised network structure (23) to the depth (17; 24, 25) thereof being between 500 : 1 and 5 : 1.

2. Article as claimed in claim 1, characterised in that between 50 % and 90 %, preferably between 60 % and 80 %, of the part surface (65 to 68) is recessed.

3. Article as claimed in claim 1 or 2, characterised in that if the anti-friction layer (9) is viewed from above, at least 50 % of the recesses and/or raised portions are surrounded on all sides by the network-type structure (23).

4. Article as claimed in one or more of the preceding claims, characterised in that the material (10) of the anti-friction layer (9) is made from at least one natural and/or synthetic latex.

5. Article as claimed in one or more of the preceding claims, characterised in that the material (10) contains a polyurethane and/or polyacrylate and/or polymethacrylate and/or polysiloxane or similar products.

6. Article as claimed in claim 5, characterised in that the anti-friction layer (9) contains 0 % by weight to 30 % by weight of polyurethane, 1 % by weight to 40 % by weight of polyacrylate, 1 % by weight to 20 % by weight of polysiloxane, 0 % by weight to 40% by weight of fillers.

7. Article as claimed in claim 6, characterised in that the filler is provided in the form of a pulverised or powdered material such as chalk, lime, corn starch, silicon dioxide and/or gypsum.

8. Article as claimed in one or more of the preceding claims, characterised in that the polymer material (10) contains highly hydrophilic acrylic or methacrylic acid and/or acrylate and/or methacrylate groups.

9. Article as claimed in one or more of the preceding claims, characterised in that the material (10) has a high surface adhesion and is preferably not cross-linked.

10. Article as claimed in one or more of the preceding claims, characterised in that at a silicon is applied at least to the regions of the anti-friction layer (9) which have a roughened surface.

11. Article as claimed in claim 10, characterised in that the quantity of silicon, at least on the surface of the part-regions (7, 18), is between 0.05 g/m² and 3 g/m², preferably 0.25 g/m².

12. Article as claimed in one or more of the preceding claims, characterised in that the anti-friction layer or layers (9) is or are applied to the inner surface (12) of the sheath-like base layer (3).

13. Article as claimed in one or more of the preceding claims, characterised in that the anti-friction layer or layers (9) is or are applied to the external surface (12) of the sheath-like base layer (3).

14. Article as claimed in one or more of the preceding claims, characterised in that when there is a relative displacement between the article (1) and the skin of a user, the imparted roughness depth in part regions is smaller.

15. Article as claimed in one or more of the preceding claims, characterised in that at least one layer of the base layer (3) is made from a latex product (2) containing a polymer (5), e.g. a natural or synthetic latex.

16. Article as claimed in one or more of the preceding claims, characterised in that the anti-friction layer (9) is disposed on a base layer (3) consisting of at least one layer which is cross-linked.

17. Article as claimed in claim 15, characterised in that the latex product (2) is cross-linked beforehand.

18. Article as claimed in one or more of the preceding claims, characterised in that the base layer (3) comprises an aqueous solution of a coagulant containing calcium nitrate, polyethylene glycol and a water-soluble silicon or chalk and a natural latex mixture cross-linked beforehand.

19. Article as claimed in one or more of the preceding claims, characterised in that the base layer (3) comprises in particular a synthetic latex cross-linked beforehand, e.g. chloroprene latex, NBR latex, X-NBR latex or SBR-latex.

20. Article as claimed in one or more of the preceding claims, characterised in that the surface of the base layer (3) is chlorinated.

21. Article as claimed in claim 20, characterised in that the peripheral regions of the surface deformations raised due to the chlorination process project beyond the adjacent surface zones by a height of 0.5 µm to 10 µm, preferably < 1 µm.

22. Article as claimed in one or more of the preceding claims, characterised in that it is a rubber glove.

23. Article as claimed in one or more of the preceding claims, characterised in that it is a medical glove.

24. Article as claimed in one or more of the preceding claims, characterised in that the mean roughness depth is between 0.5 µm and 100 µm, preferably between 1 µm and 40 µm.

25. Method of manufacturing a flexible article (1), for example a glove (4), a condom, in which a base (3) layer is made from a synthetic material and/or rubber, characterised in that at least a part region (7, 18) of its surface is provided with a coating of an anti-friction layer (9) which is then immediately dried on for 10 sec. to 600 sec., after which the anti-friction layer (9) is treated with water at a temperature of between 40 °C and 100 °C or is rinsed with hot water so that the water content of the anti-friction layer (9) is reduced by approximately 40 % to 70 % as a result, and in which a roughness having recesses is produced in the region of the surface (12) of the anti-friction layer (9) remote from the base layer (3), the ratio of mean distances (15, 16) in between the recurring recesses relative to a raised network structure to the depth (24, 25) thereof being between 500:1 and 5:1.

26. Method as claimed in claim 25, characterised in that drying is effected by means of hot air.

27. Method as claimed in claim 25 or 26, characterised in that the water temperature is between 60 °C and 80 °C.

28. Method as claimed in one of claims 25 to 27, characterised in that the water content is reduced by 50 % to 60 %.

29. Method as claimed in one of claims 25 to 28, characterised in that immediately after the time when the article (1) is drained after rinsing with hot water, the article (1) is exposed to infrared radiation.

30. Method as claimed in claim 29, characterised in that radiation bodies used to emit the infrared radiation, e.g. tiles, are at a temperature of between 350 °C and 700 °C.

31. Method as claimed in one of claims 29 or 30, characterised in that the duration of exposure to infrared radiation is between 1 and 5 minutes.

32. Method as claimed in one of claims 25 to 31 , characterised in that the draining time between rinsing the article (1) with hot water and exposing it to infrared radiation is between 10 and 180 seconds, preferably between 20 and 50 seconds.

33. Method as claimed in one of claims 25 to 32, characterised in that hot air is applied to the article (1) during the draining time.

34. Method as claimed in one of claims 26 to 33, characterised in that the coagulant solution of the latex product (2) and/or anti-friction layer (9) is dried on by applying hot air to transform its surface from the liquid into the gel or solid state.

35. Method as claimed in claim 34, characterised in that the surface of the latex product (2) and/or the anti-friction layer (9) is dried on at a temperature of 70 °C to 140 °C, preferably 90 °C to 110 °C.

36. Method as claimed in one of claims 34 or 35, characterised in that the latex product (2) and the anti-friction layer (9) is dried on for between 10 and 80 seconds, preferably 15 to 60 seconds.

37. Method as claimed in one of claims 25 to 36, characterised in that the temperature of the hot water with which the anti-friction layer (9) is rinsed is between 40 °C and 95 °C, preferably between 70 °C and 90 ° C, and the treatment lasts for between 15 seconds and 8 minutes, preferably between 30 seconds and 4 minutes.

38. Method as claimed in one of claims 25 to 37, characterised in that the anti-friction layer (9) is made from a dispersion of at least one polymer material (10).

39. Method as claimed in claim 38, characterised in that the dispersion comprises a mixture of between 0 % by weight and 30 % by weight of polyurethane-, between 1 % by weight and 40 % by weight of polyacrylate-, between 1 % by weight and 20 % by weight of polysiloxane-dispersion, between 0 % by weight and 10 % by weight of fillers, the remaining proportion being water.

40. Method as claimed in claim 39, characterised in that the filler is a pulverised or powdered material such as chalk, lime, corn starch, silicon dioxide and/or gypsum.

41. Method as claimed in one of claims 38 to 40, characterised in that the viscosity of the dispersion of the anti-friction layer (9) is between 5 mPa.s and 30 mPa.s, preferably between 7 mPa.s and 11 mPa.s.

42. Method as claimed in one of claims 25 to 41, characterised in that the roughness is imparted by hollowing out textured surfaces on the base layer (3).

43. Method as claimed in one of claims 25 to 42, characterised in that the recesses are produced by pressing in micro-balls or by sand-blasting.

44. Method as claimed in one of claims 25 to 43, characterised in that an anti-friction substance, in particular a silicon, is applied to the roughened surfaces of the part regions.

45. Method as claimed in one of claims 25 to 44, characterised in that the mean roughness depth is between 0.5 µm and 100 µm.

## Revendications

1. Article (1) en une couche de support (3) en un caoutchouc flexible et/ou en matière synthétique, qui est réalisé, au moins avec une zone partielle (7) de la surface, pour le contact avec ou l'application à la peau humaine, notamment un gant (4), un condom ou analogue, qui est pourvu dans une zone partielle (7, 18) de la surface d'une couche de glissement (9) en matériau polymère (10), caractérisé en ce qu'au moins une face partielle (65 à 68) de la surface (12) est réalisée comme creux et/ou surélévations en matériau polymère (10), où la couche de glissement (9) est fixée sur la couche de support (3), et le rapport des écarts moyens (15, 16) entre les surélévations respectivement les creux se répétant en face d'une structure surélevée en forme de filet (23) à leur profondeur (17 ; 24, 25) est compris entre 500 : 1 et 5 : 1.

2. Article selon la revendication 1, caractérisé en ce qu'entre 50 % et 90 %, de préférence 60 % à 80 % de la face partielle (65 à 68) sont réalisés en creux.

3. Article selon la revendication 1 ou 2, caractérisé en ce qu'au moins 50 % des creux et/ou surélévations, en vue de dessus de la couche de glissement (9), sont entourés de tout côté par la structure (23) en forme de filet.

4. Article selon l'une ou plusieurs des revendications précédentes, caractérisé en ce que le matériau (10) de la couche de glissement (9) est formé par au moins un latex naturel et/ou de synthèse.

5. Article selon l'une ou plusieurs des revendications précédentes, caractérisé en ce que le matériau (10) contient une polyuréthanne et/ou un polyacrylate et/ou des polyméthacrylates et/ou des polysiloxanes ou des produits comparables.

6. Article selon la revendication 5, caractérisé en ce que la couche de glissement (9) contient 0 % en poids jusqu'à 30 % en poids de polyuréthanne, 1 % en poids jusqu'à 40 % en poids de polyacrylate, 1 % en poids jusqu'à 20 % en poids de polysiloxane, 0 % en poids jusqu'à 40 % en poids de matières de remplissage.

7. Article selon la revendication 6, caractérisé en ce que la matière de remplissage, sous forme pulvérulente ou de poudre, est formée par des matériaux comme la craie, la chaux, l'amidon de maïs, le dioxyde de silicium et/ou le gypse.

8. Article selon l'une ou plusieurs des revendications précédentes, caractérisé en ce que le matériau polymère (10) contient des groupes d'acide acrylique et/ou méthacrylique et/ou d'acrylate et/ou de méthacrylate fortement hydrophiles.

9. Article selon l'une ou plusieurs des revendications précédentes, caractérisé en ce que le matériau (10) présente une forte adhésion superficielle et est de préférence non réticulé.

10. Article selon l'une ou plusieurs des revendications précédentes, caractérisé en ce qu'une silicone est appliquée au moins sur les zones de la couche de glissement (9) qui sont rendues rugueuses.

11. Article selon la revendication 10, caractérisé en ce que la quantité de silicone, au moins sur la surface des zones partielles (7, 18), est comprise entre 0,05 g/m² à 3 g/m² , et qu'elle est de préférence de 0,25 g/m².

12. Article selon l'une ou plusieurs de revendications précédentes, caractérisé en ce que la ou les couches de glissement (9) sont appliquées sur la surface interne (12) de la couche de support (3) semblable à une gaine.

13. Article selon l'une ou plusieurs des revendications précédentes, caractérisé en ce que la ou les couches de glissement (9) sont appliquées sur la surface externe (12) de la couche de support (3) semblable à une gaine.

14. Article selon l'une ou plusieurs des revendications précédentes, caractérisé en ce que la profondeur de rugosité moyennée est plus petite dans des zones partielles d'un mouvement relatif plus restreint entre l'objet (1) et la peau d'un utilisateur.

15. Article selon l'une ou plusieurs des revendications précédentes, caractérisé en ce qu'au moins une couche de la couche de support (3) est formée par un produit en latex (2) qui comprend un polymère (5), par exemple un latex naturel et/ou de synthèse.

16. Article selon l'une ou plusieurs des revendications précédentes, caractérisé en ce que la couche de glissement (9) est disposée sur une couche de support (3) constituée d'au moins une couche qui est légèrement réticulée.

17. Article selon la revendication 15, caractérisé en ce que le produit en latex (2) est préalablement réticulé.

18. Article selon l'une ou plusieurs des revendications précédentes, caractérisé en ce que la couche de support (3) est constituée par une solution aqueuse d'un coagulant, qui contient du nitrate de calcium, du polyéthylène-glycol et une silicone ou craie soluble dans l'eau, et qu'un mélange de latex naturel préalablement réticulé est formé.

19. Article selon l'une ou plusieurs des revendications précédentes, caractérisé en ce que la couche de support (3) présente un latex de synthèse notamment préalablement réticulé, par exemple un latex de cloroprène, un latex NBR, un latex X-NBR ou un latex SBR.

20. Article selon l'une ou plusieurs des revendications précédentes, caractérisé en ce que la couche de support (3) est chlorée à sa surface.

21. Article selon la revendication 20, caractérisé en ce que les zones de bord surélevées, remontées par le chlorage, des déformations de surface font saillie sur les zones de surface avoisinantes selon une hauteur de 0,5 µm jusqu'à 10 µm, de préférence de ≤ 1 µm.

22. Article selon l'une ou plusieurs des revendications précédentes, caractérisé en ce que celui-ci est réalisé sous forme de gant en caoutchouc.

23. Article selon l'une ou plusieurs des revendications précédentes, caractérisé en ce que celui-ci est réalisé sous forme de gant médical.

24. Article selon l'une ou plusieurs des revendications précédentes, caractérisé en ce que la profondeur de rugosité moyennée est comprise entre 0,5 µm et 100 µm, de préférence entre 1 µm et 40 µm.

25. Procédé de fabrication d'un article flexible (1), par exemple d'un gant (4), d'un condom, dans lequel est fabriquée une couche de support (3) en matière synthétique et/ou en caoutchouc, caractérisé en ce qu'il est appliqué sur celle-ci par immersion, sur au moins une zone partielle (7, 18) de sa surface, une couche de glissement (9), qui est séchée directement ensuite pendant 10 s à 600 s, à la suite de quoi la couche de glissement (9) est traitée avec de l'eau d'une température entre 40 °C et 100°C ou est rincée avec l'eau chaude de telle sorte qu'ensuite, la teneur en eau de la couche de glissement (9) diminue d'environ 40 % à 70 %, et où est produit au voisinage de la surface (12) de la couche de glissement (9) éloignée de la couche de support (3) une rugosité avec des creux, où le rapport d'écarts moyens (15, 16) entre des creux se répétant en face d'une structure surélevée (23) en forme de filet, à leur profondeur (24, 25) est compris entre 500 : 1 et 5 : 1.

26. Procédé selon la revendication 25, caractérisé en ce que l'application par séchage a lieu avec de l'air chaud.

27. Procédé selon la revendication 25 ou 26, caractérisé en ce que la température de l'eau est comprise entre 60°C et 80°C.

28. Procédé selon l'une des revendications 25 à 27, caractérisé en ce que la teneur en eau est diminuée de 50 % à 60 %.

29. Procédé selon l'une des revendications 25 à 28, caractérisé en ce que, directement à la suite du temps d'assèchement après le rinçage de l'article (1) avec l'eau chaude, l'article (1) est exposé à un rayonnement infrarouge.

30. Procédé selon la revendication 29, caractérisé en ce que le corps de rayonnement pour l'émission du rayonnement infrarouge, par exemple des carreaux de faïence, présente une température comprise entre 350°C et 700°C.

31. Procédé selon l'une des revendications 29 ou 30, caractérisé en ce que la durée de l'exposition aux rayons infrarouges est comprise entre 1 et 5 minutes.

32. Procédé selon l'une des revendications 25 à 31, caractérisé en ce que le temps d'assèchement entre le rinçage de l'article (1) avec l'eau chaude et l'action du rayonnement infrarouge est compris entre 10 et 180 secondes, de préférence entre 20 et 50 secondes.

33. Procédé selon l'une des revendications 25 à 32, caractérisé en ce que l'article (1), pendant le temps d'assèchement, est soumis à de l'air chaud.

34. Procédé selon l'une des revendications 26 à 33, caractérisé en ce que lors du séchage, effectué avec de l'air chaud, de la solution de coagulation de l'article en latex (2) et/ou de la couche de glissement (9), la surface de celle-ci est amenée de l'état liquide à l'état en forme de gel respectivement solide.

35. Procédé selon la revendication 34, caractérisé en ce que le séchage de la surface de l'article en latex (2) et/ou de la couche de glissement (9) a lieu à une température de 70°C à 140°C, de préférence de 90°C à 110°C.

36. Procédé selon l'une des revendications 34 ou 35, caractérisé en ce que l'opération de séchage de l'article en latex (2) et de la couche de glissement (9) dure entre 10 et 80 secondes, de préférence 15 à 60 secondes.

37. Procédé selon l'une des revendications 25 à 36, caractérisé en ce que la température de l'eau chaude pour le rinçage de la couche de glissement (9) est comprise entre 40°C et 95°C, de préférence entre 70°C et 90°C, et la durée de traitement est de 15 secondes à 8 minutes, de préférence de 30 secondes à 4 minutes.

38. Procédé selon l'une des revendications 25 à 37, caractérisé en ce que la couche de glissement (9) est formée par une dispersion à partir d'au moins un matériau polymère (10).

39. Procédé selon la revendication 38, caractérisé en ce que la dispersion est formée par un mélange de 0 % en poids à 30 % en poids de dispersion de polyuréthanne, de 1 % en poids à 40 % en poids de dispersion de polyacrylate, de 1 % en poids à 20 % en poids de dispersion de polysiloxane, de 0 % en poids à 10 % en poids de matières de remplissage, le reste étant de l'eau.

40. Procédé selon la revendication 39, caractérisé en ce que la matière de remplissage, sous forme pulvérulente ou de poudre, est constituée par des matériaux comme la craie, la chaux, l'amidon de maïs, le dioxyde de silicium et/ou le gypse.

41. Procédé selon l'une des revendications 38 à 40, caractérisé en ce que la viscosité de la dispersion de la couche de glissement (9) représente entre 5 mPa.s jusqu'à 30 mPa.s, de préférence 7 mPa.s à 11 mPa.s.

42. Procédé selon l'une des revendications 25 à 41, caractérisé en ce que la rugosité est formée par les faces de formage à la couche de support (3).

43. Procédé selon l'une des revendications 25 à 42, caractérisé en ce que les creux sont réalisés par enfoncement de microbilles ou par décapage au sable.

44. Procédé selon l'une des revendications 25 à 43, caractérisé en ce qu'un agent de glissement, notamment une silicone, est appliqué sur les surfaces rendues rugueuses des zones partielles.

45. Procédé selon l'une des revendications 25 à 44, caractérisé en ce que la profondeur de rugosité moyennée se situe entre 0,5 µm et 100 µm.
